# EUROPÄISCHE PATENTSCHRIFT

(11) **EP 2 338 880 B1**
(45) Veröffentlichungstag und Bekanntmachung des Hinweises auf die Patenterteilung: **04.06.2014**
(21) Anmeldenummer: 10195558.1
(22) Anmeldetag: 17.12.2010
(51) Int. Cl.: C07D 209/46, B29C 45/00, B29C 47/00, C08G 4/00, C08L 69/00, C08G 64/12

(54) **Mehrschichtige Erzeugnisse enthaltend ein Polycarbonat mit verbesserten thermischen und mechanischen Eigenschaften sowie reduziertem thermischen Ausdehnungskoeffizienten**
Multilayer products comprising a polycarbonate with improved thermal and mechanical characteristics and reduced thermal expansion coefficients
Produits multicouches contenant du polycarbonate doté de propriétés thermiques et mécaniques améliorées et d'un coefficient d'extension thermique réduit

(30) Priorität: 21.12.2009 DE 102009059771
(43) Veröffentlichungstag der Anmeldung: 29.06.2011
(73) Patentinhaber: Bayer Intellectual Property GmbH, 40789 Monheim (DE)
(72) Erfinder: Heuer, Helmut-Werner, 47829, Krefeld (DE); Wehrmann, Rolf, 47800, Krefeld (DE)
(74) Vertreter: BIP Patents

(56) Entgegenhaltungen:
- WO-A1-2008/121149
- ADAMCZYK MACIEJ ET AL: "A PRACTICAL METHOD FOR THE SYNTHESIS OF PHENOLPHTHALEIN SPIROLACTAMS", ORGANIC PREPARATIONS AND PROCEDURES INTERNATIONAL, ORGANIC PREPARATION AND PROCEDURES CO., NEWTON HIGHLANDS, MA, US, Bd. 33, Nr. 1, 1. Januar 2001 (2001-01-01) , Seiten 95-100, XP009083715, ISSN: 0030-4948
- LOEWE S: "STUDIES ON THE LAXATIVE ACTIVITY OF TRIPHENYLMETHANE DERIVATIVES: I. Relationship between Structure and Activity of Phenolphthalein Congeners", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, Bd. 94, Nr. 3, 1. November 1948 (1948-11-01), Seiten 288-298, XP008084542, ISSN: 0022-3565
- MORGAN: "Linear condensation polymers from phenolphthalein and related compounds", JOURNAL OF POLYMER SCIENCE PART A: GENERAL PAPERS, Bd. 2, Nr. 1, 1964, Seiten 437-459, XP002623513,

## Beschreibung

Die vorliegende Erfindung betrifft mehrschichtige Erzeugnisse enthaltend weinigstens ein thermoplastisches Hoch-Tg-Polycarbonate, die sich durch verbesserte thermische Eigenschaften sowie verbesserte mechanische Eigenschaften, insbesondere durch verringerte thermische Ausdehnung, auszeichnen. Weiterhin betrifft die vorliegende Erfindung ein Verfahren zur Herstellung dieser mehrschichtigen Erzeugnisse. Insbesondere betrifft diese Erfindung mehrschichtige Erzeugnisse enthaltend wenigstens ein Polycarbonate mit einer Struktureinheit, die sich von Phthalimid der Formel (I) ableitet, sowie ein Verfahren zur Herstellung dieser mehrschichtigen Erzeugnisse.

(Co)polycarbonate gehören zur Gruppe der technischen Thermoplaste. Sie finden vielseitige Anwendungen im Elektro- und Elektronikbereich, als Gehäusewerkstoff von Leuchten und bei Anwendungen, bei denen sowohl besondere thermische und mechanische Eigenschaften als auch hervorragende optische Eigenschaften gefordert sind, beispielsweise Anwendungen im Automobilbereich, Kunststoffabdeckungen, Streuscheiben oder Lichtleitelemente sowie Lampenabdeckungen, Lampeneinfassungen (bezels). Reflektoren bzw. Subreflektoren aus einem Thermoplasten durch Spritzguss mit hervorragender Oberflächenqualität hergestellt, wären eine sehr interessante Ergänzung dieser Anwendungen für (Co)polycarbonate.

Bei diesen Anwendungen werden praktisch immer die guten thermischen Eigenschaften wie Vicat-Temperatur (Wärmeformbeständigkeit) und Glastemperatur zwingend benötigt. Auch eine gute Metallhaftung, beispielsweise für Aluminium, ist für einige Anwendungen unabdingbar. Gleichzeitig sind hervorragende optische Eigenschaften von höchster Bedeutung. Unberücksichtigt blieben bisher mechanische Eigenschaften hinsichtlich thermischer Ausdehnung des amorphen Polycarbonats bei gleichbleibenden thermischen Eigenschaften, wie der lineare thermische Ausdehnungskoeffizient.

Thermoplastische Kunststoffe, aus denen durch Spritzgießen und anschließende Metallisierung (Vakuumbeschichtung, zumeist mit Aluminium) lichtreflektierende Bauteile hergestellt werden, sind bekannt. Solche Bauteile sind z. B. Scheinwerferreflektoren für Automobile. Neben den früher ausnahmslos verwendeten Paraboloid-Scheinwerfern wurden zwei hinsichtlich Lichtausnutzung und Platzbedarf optimierte Grundtypen entwickelt, die Projektions- (Ellipsoid, Polyellipsoid) und die Freiflächen-Scheinwerfer. Da die Abdeckscheiben insbesondere von Freiflächen-Scheinwerfern in Folge der optimierten Lichtausnutzung und -verteilung dieses Reflektortyps meist ohne Profilierung gestaltet werden können, sind heute Klarsichtscheiben aus Polycarbonat im Einsatz. Dies erhöht die Anforderungen an die Oberflächengüte der von außen gut Subreflektor, Blendrahmen), wobei die Dimensionsbeständigkeit in der Wärme, ein geringes Ausgasen um Blasenbildung zu vermeiden, die mechanische Festigkeit, eine einfache Verarbeitung und geringe Fertigungstoleranzen weiterhin wichtig sind.

Bisher werden Scheinwerferreflektoren entweder aus Blech oder metallisierten Spritzgussteilen aus Duroplast (Bulk Molding Compounds, BMC) hergestellt. Gefordert sind hier gute Maßhaltigkeit sowie Temperaturbeständigkeit.

Scheinwerferreflektoren können auch in den eigentlichen, im Wesentlichen eine Paraboloidform aufweisenden Reflektor und einen mehr oder weniger von der Paraboloidform abweichenden Subreflektor unterteilt werden. Der Reflektor ist das eigentliche, das Licht zur gewünschten Beleuchtung gezielt reflektierende Bauteil, das normalerweise in unmittelbarer Umgebung der das Licht erzeugenden Glühlampe angeordnet ist. Die Lampe bzw. Glühbirne oder eine diesen entsprechende Lichtquelle erzeugt neben dem Licht auch Wärme, so dass der Reflektor je nach Konstruktion einer Betriebstemperatur von etwa 180-220°C ausgesetzt sein kann. Aus diesem Grunde ist es notwendig, Materialien mit einem möglichst geringen linearen thermischen Ausdehnungskoeffizienten zur Verfügung zu stellen. Dieses Material soll möglichst durch Spritzgußtechnologie verarbeitet werden können und preiswert sein.

Zudem sollten die Reflektoren in einem Temperaturbereich von -50°C bis 220°C formstabil sein, d.h. das Ausdehnungs- und Schwindungsverhalten muss möglichst isotrop sein, damit - zumindest bei den Reflektoren - die Lichtausbeute bzw. Lichtbündelung nicht beeinträchtigt wird. Bevorzugt weisen die Metallschichten ein im Wesentlichen gleiches Ausdehnungs- und Schwindungsverhalten wie die Reflektoren auf, so dass die Zug- bzw. Schubbeanspruchung der Reflexionsschichten möglichst klein ist. Dadurch wird zusätzlich die Gefahr einer Rissbildung oder Stauchung in den Reflexionsschichten reduziert.

Bisher wurden zum Herstellen von Reflektoren zumeist Duroplaste, seltener auch Thermoplaste eingesetzt. Von den letzteren weisen die hauptsächlich verwendeten amorphen Thermoplaste, z.B. Polyetherimid (PEI), Polyamidimid (PAI) oder Polysulfone, z.B. Polyethersulfon (PES) bzw. Polysulfon (PSU) bzw. Polyphenylenethersulfon (PPSU), eine hohe bis sehr hohe Glasübergangstemperatur (Tg) auf (vgl. z.B. das PAI TORLON^{®} der Firma Solvay Advanced Polymers). Diese amorphen Hoch-Tg-Thermoplaste können ohne Füllstoffe zum Erzeugen von Reflektor-Rohlingen mit einer hervorragenden Oberflächenglätte verwendet werden. Die Reflektor-Rohlinge können direkt metallisiert werden. Nachteilig für eine Massenproduktion ist allerdings der zum Teil sehr hohe Preis der genannten amorphen Hoch-Tg-Thermoplaste. Zudem gestaltet sich die Verarbeitung dieser Hoch-Tg-Thermoplaste zum Teil als schwierig.

Für Scheinwerferreflektoren kommen seit einiger Zeit hauptsächlich Bulk Molding Compounds (BMC) zur Anwendung. Dabei handelt es sich um ein Faser-Matrix-Halbzeug. Es besteht zumeist aus Kurz-Glasfasern und einem Polyester- oder Vinylesterharz, andere Verstärkungsfasern oder Harzsysteme sind möglich. BMC wird im Heißpressverfahren verarbeitet, was kurze Taktzeiten ermöglicht. Die BMC Masse wird dazu zentral in ein beheiztes, geteiltes Werkzeug eingelegt. Beim Schließen verteilt sich das BMC im Werkzeug. Durch die kurzen Faserlängen können beim Pressen auch dünne Rippen und Wanddicken gefüllt werden. Es besteht jedoch die Gefahr, dass sich an Engstellen das BMC entmischt. Dies geschieht dann, wenn eine Engstelle mit Fasern verstopft, so dass nur noch das Harz weiterfließen kann. Die einzelnen Verstärkungsfasern orientieren sich in der Regel in Strömungsrichtung, so dass lokal stark orientierte Fasern auftreten können. In Spezialverfahren kann BMC, bei entsprechend kleinen Faserlängen, auch im Spritzgussverfahren verarbeitet werden.

Eine typische Anwendung für Duroplaste (BMC) sind PKW-Scheinwerfer, genauer die Reflektoren des Scheinwerfers. Hier kommt die gute Maßhaltigkeit und Temperaturbeständigkeit zum Tragen. Der Prozess ähnelt sehr stark dem Elastomer-Spritzgießen. Die Zykluszeiten sind bei der Duroplastverarbeitung in der Regel bei Wanddicken bis ca. 4 mm länger als bei Thermoplasten. Dadurch verlieren Duroplaste im Wirtschaftlichkeitsvergleich meist gegenüber den Thermoplasten, wenn die guten elektrischen oder mechanischen Eigenschaften nicht benötigt werden.

Die Füllstoffe haben dabei überwiegend die Aufgabe das BMC kostengünstiger herzustellen, indem Faser- und Harzvolumen durch billigere Füllstoffe ersetzt wird. Je nach gewünschten Eigenschaften, zum Beispiel erhöhter Flammschutz oder niedriger Schrumpf, werden Zusatzstoffe zugesetzt. So erhöht zum Beispiel Magnesiumoxid die Plastizität und Kaolin die Säurebeständigkeit.

In der Beleuchtungseinheit treten naturgemäß die höchsten Temperaturen auf. Daher wurden bisher die Reflektoren entweder aus Blech, aus Duroplasten, wie BMC oder aus metallisierten, spritzgegossenen amorphen Hoch-Tg-Thermoplasten (PEI, PSU, PES) hergestellt. Die hohen Toleranzanforderungen, gekoppelt mit der für die Metallisierung erforderlichen Oberflächengüte der Spritzgussteile, wurden bisher hauptsächlich von ungefüllten amorphen Hoch-Tg-Thermoplasten oder lackierten Duroplasten erfüllt.

Ein Beispiel für einen der genannten Hoch-Tg-Thermoplaste ist das Polyethersulfon ULTRASON E^{®} von BASF Ludwigshafen, Deutschland (mit einer Irisiertemperatur von 212°C), wie es in der nachfolgend zitierten Zeitschrift beschrieben ist. Im Zuge der fortschreitenden Komplexizitätsreduzierung findet zur Zeit eine zunehmende Integration von Scheinwerferkomponenten zu hoch entwickelten Beleuchtungssystemen statt, die höhere Materialanforderungen erwarten lassen (J. Queisser, M. Geprägs, R. Blum und G. Ickes, Trends bei Automobilscheinwerfern, Kunststoffe 3/2002, Hanser Verlag, München).

Aus dem Stand der Technik sind zudem Zusammensetzungen bekannt, die einen fibrillären, anorganischen Füllstoff (vgl. EP 0 863 180) und einen zusätzlichen körnigen anorganischen Füllstoff umfassen (EP 1 312 647 oder EP 0 585 056) oder die nur einen körnigen, anorganischen Füllstoff umfassen (EP 0 913 421). Unter dem Namen MINLON^{®} (E.I. du Pont de Nemours & Co., Wilmington, USA) ist ein Material zur Herstellung von Strassenbeleuchtungsreflektoren bekannt. Bei dem genannten Produkt handelt es sich um das teilkristalline Nylon 66 (PA 66), welches neben einem Hitzestabilisator auch 36-40% klassische Mineralstoffe umfasst. Allerdings erscheint dieses Material von der Oberflächengüte her zumindest für Fahrzeugfahrbeleuchtungen nicht geeignet. Als weiterer Nachteil wird auch hier das erhebliche Verlängern der Zykluszeiten beim Spritzgießen mit solchen Zusammensetzungen gegenüber amorphen Polymeren angesehen.

Eine weitere Anforderung betrifft die Oberflächengüte der zu beschichtenden (meist gekrümmten) Kunststoffoberfläche. Speziell bei Reflektoren, bei welchen die Lichtausbeute essentiell ist, muss eine möglichst homogene, sehr glatte, hochglänzende Oberfläche für die Beschichtung bereitgestellt werden. Schlecht fließende oder zu früh erstarrende Kunststoffe bzw. eine Zugabe von Füllstoffen führen in der Spritzgußform oft zu einem rauen, matten oder unregelmäßigen Abdruck, gemessen an den extrem hohen Anforderungen einer spiegelglatten Oberfläche, selbst wenn die entsprechende Oberfläche des formgebenden Werkzeugs hochglanzpoliert ist.

Aus dem Stand der Technik sind weitere Zusammensetzungen ohne Füllstoffe bekannt. Diese erreichen aber ebenfalls nur ungenügende Tg-Werte von weniger als 175°C (vgl. z.B. EP 0 313 436, EP 0 553 581 und US 4,898,896). Zu dieser Kategorie von für die geplante Verwendung ungenügenden Polymere gehören auch Polyarylamide wie IXEF^{®} 2057 (Solvay Advanced Polymers), Polyarylate, Polybutylen-Therephthalat (PBT, wie z.B. das ARNITE^{®} TV4 220 von DSM).

Die im europäischen Patent EP 0 725 101 B2 offenbarten transparenten, farblosen und amorphen Homopolyamide weisen eine Glastemperatur von ca. 157°C auf und sind allenfalls für die Herstellung von Subreflektoren, aber nicht für die Herstellung von Lichtreflektierbauteilen geeignet, die für Betriebstemperaturen von mindestens 200°C ausgelegt sind.

Aus dem Patent US 6,355,723 B1 sind spritzgegossene Reflektoren aus amorphen Thermoplasten, wie Polyetherimiden, Polyarylethern, Polyethersulfonen, Polysulfonen, Polycarbonaten, Polyestercarbonaten, Polyarylaten, Polyamiden, Polyestern und Einzelphasengemischen solcher Thermoplaste bekannt. Diese Reflektoren können direkt mit einer Metallschicht versehen werden und eine Thermoplaste bekannt. Diese Reflektoren können direkt mit einer Metallschicht versehen werden und eine Glasübergangstemperatur (Tg) von mindestens 170°C bis 200°C aufweisen. Um auffällige Oberflächenfehler vor dem Metallisieren der Reflektoroberfläche mittels Sichtinspektion einfacher feststellen zu können und um unerwünschte Lichteffekte durch nicht-metallisierte Teile der Reflektoren zu unterbinden, sind alle diese Reflektoren mittels Beimischen von Farbstoffen schwarz eingefärbt.

Die im Stand der Technik vorbeschriebenen Polycarbonate bzw. Copolycarbonate haben jedoch durch ihre zu hohen Ausdehnungskoeffizienten den Nachteil, dass sie beim Einsatz als metallisiertes Bauteil bei z. B. Hochtemperaturanwendungen als Reflektor nur beschränkt oder gar nicht geeignet sein können.

Für die Anwendung flexibler Substrate für Displayanwendungen, beispielsweise LCD- bzw. OLED-Displays, werden Polymermaterialien mit sehr guten optischen und thermischen Eigenschaften benötigt. So ist für die Erzeugung der Thin Film Transistor (TFT)-Elemente auf den Substraten, beispielsweise durch die a-Si:H-Methode, eine ausreichend hohe Glastemperatur des Materials notwendig. Polyethylennaphtalat (PEN) zeigt zwar einen geringen thermischen Ausdehnungskoeffizienten, weist jedoch nur eine geringe Glastemperatur von 120 °C auf und wirkt doppelbrechend. Polyarylate (PAR) zeigen zwar eine hohe Glastemperatur (215 °C) und optisch isotrope Erscheinung, sind allerdings sehr kostspielige Materialien. Dies gilt auch für Polyethersulfon (PES, Tg = 220 °C). Polyimide haben die höchste Glastemperatur (360 °C), neben sehr geringen thermischen Ausdehnungskoeffizienten, erscheinen jedoch orange bis braun im optischen Aussehen. Die Kosten für diese Materialien sind ebenfalls sehr hoch.

Aus WO 2008/121149 A1 sind Polycarbonate, welche sich von 2-Hydrocarbyl-3,3-bis(4-hydroxyaryl)phtalimidin-Monomeren ableiten, sowie ein Verfahren zur Herstellung dieser Monomere bekannt.

Für Anwendungen in der Displaytechnologie sind sehr gute optische Eigenschaften, wie hohe Transparenz ohne Doppelbrechung erforderlich. Ein Polymermaterial, welches zu vertretbaren Kosten bei gleichzeitiger hoher Glastemperatur, geringer thermischen Ausdenung eine sehr gute Transparenz bei isotroper optischer Erscheinung aufweist, wäre für die Displaytechnologie wünschenswert.

Es bestand daher die Aufgabe, aromatische (Co)Polycarbonate mit verringerten thermischen Ausdehnungskoeffizienten zu entwickeln, die sich gleichzeitig durch eine hervorragende Metallhaftung und guten thermischen Eigenschaften (insbesondere hohe Vicat- oder GlasTemperatur) auszeichnen, bei gleich guter Oberfläche (die für die direkte Beschichtung mit einer Metallschicht ohne zusätzlichem Vorbehandlungsschritt geeignet ist) und mit vergleichbar guter Wärmeformbeständigkeit hergestellt werden können, als mit aus dem Stand der Technik bekannten Materialien.

Die neuen Zusammensetzungen sollten außerdem eine verbesserte Fließfähigkeit zeigen.

Unter Polycarbonatzusammensetzungen (oder Blends) im Sinne dieser Anmeldung versteht man Mischungen aus zwei oder mehreren Polycarbonaten, die gegebenenfalls mit Zusatzstoffen (weitere Komponente) versehen sein können.

Überraschenderweise wurde nun gefunden, dass ein Copolycarbonat enthaltend die Struktureinheit der Formel (I), bei gleichbleibenden thermischen Eigenschaften (Vicat-Temperatur) und mechanischen sowie optischen Eigenschaften geringere thermische Ausdehnungskoeffizienten aufweist:

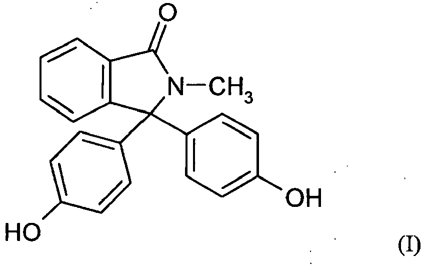

Das Phthalimids der Formel (I) kann durch Umsetzung von Phenolphtalein mit einer wässrigen Methylaminlösung in Gegenwart von N-Methylpyrrolidon hergestellt werden.

Bevorzugt wird dabei eine 20 bis 80 Gew.%-ige bevorzugt 30 bis 60%-ige besonders bevorzugt 35 bis 45%-ige Methylaminlösung, bevorzugt eine wässrige Lösung, in 3,5 bis 4,5-fachen molarem Überschuss an Phenolphtalein in Lösung, bevorzugt ein N-Methylpyrrolidon-Wasser-Gemisch, umgesetzt. Bevorzugt werden auf 4 mol Phenolphtalein 2 kg N-Methylpyrrolidon und 2 kg Wasser als Lösungsmittelgemisch verwendet.

Das N-Methylpyrrolidon wird bevorzugt bei 40 bis 60°C, besonders bevorzugt 45 bis 55°C, ganz besonders bevorzugt bei 50 °C vorgelegt und der Reaktand Phenolphtalein hinzugegeben. Nach Erhalt einer homogenen Lösung, wird das restliche Wasser des Lösungsmittelgemisches sowie die Methylamin-Lösung als weiterer Reaktand zugegeben. Die Reaktion erfolgt bevorzugt in einem Temperaturbereich von 65 bis 95 °C, bevorzugt 75 bis 90 °C und besonders bevorzugt bei 80 bis 88 °C.

Die Isolierung und Reinigung des Produktes erfolgt durch Ausfällen in einer salzsauren, wässrigen Lösung, bevorzugt 37 %-ig und anschließendes Auflösen in Natronlauge nach bekannten Methoden. Der Zyklus der Isolierung und Reinigung wird mehrmals hintereinander durchgefürt, bevorzugt 1-3 mal.

Gegenstand der Erfindung ist ein mehrschichtiges Erzeugnis enthaltend ein Substrat, das mindestens auf einer Seite eine weitere Schicht aufweist, wobei das Substrat aus wenigstens einem (Co)Polycarbonat oder wenigstens einem Blend enthaltend ein oder mehrere (Co)Polycarbonate und ein oder mehrere thermoplastische Polymere hergestellt wird, dadurch gekennzeichnet, dass die (Co)Polycarbonat(e) Bisphenole der Formel (Ia) als wiederkehrende Monomereinheit im Polymer 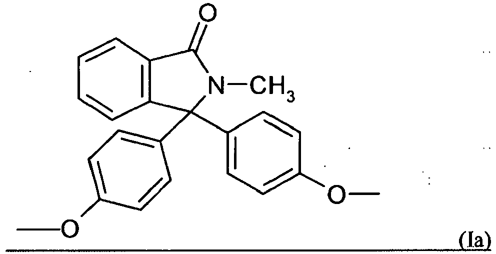 enthalten. Gegenstand der Erfindung sind ebenfalls Verfahren zur Herstellung der mehrschichtigen Erzeugnisse

Die erfindungsgemäß zu verwendenden Polycarbonate werden dadurch erhalten, dass der erfindungsgemäße Bisphenolbaustein mit gegebenenfalls weiteren Bisphenolbausteinen im Phasengrenzflächenverfahren bzw. dem Schmelzeumesterungsverfahren zu höhermolekularen Copolycarbonaten aufkondensiert werden.

Die erfindungsgemäß zu verwendenden (Co)Polycarbonate basieren auf Bisphenolen der allgemeinen Formeln (Ia) als wiederkehrende Monomereinheit:

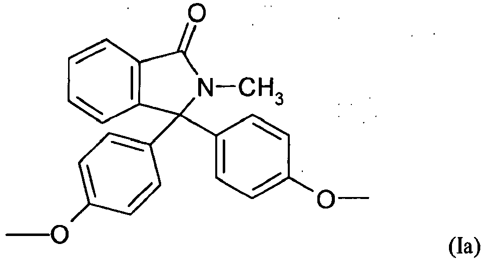

Im Falle von (Co)Polycarbonaten können neben einem oder mehreren Diphenolen der Formel (I), als weitere Monomereinheit Bisphenole der Formel (II) enthalten sein: 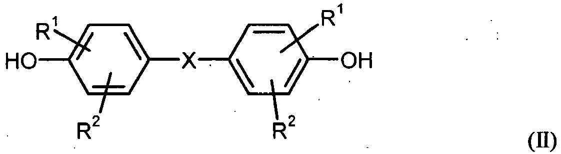 in der
- R1 und R2: unabhängig voneinander für H, C1-C18-Alkyl-, C1-C18-Alkoxy, Halogen wie Cl oder Br oder für jeweils gegebenenfalls substituiertes Aryl oder Aralkyl, bevorzugt für H oder C1-C12-Alkyl, besonders bevorzugt für H oder C1-C8-Alkyl und ganz besonders bevorzugt für H oder Methyl stehen, und
- X: für eine Einfachbindung, -SO₂-, -CO-, -O-, -S-, C₁- bis C₆-Alkylen, C₂- bis C₅-Alkyliden oder C₅- bis C₆- Cycloalkyliden, welches mit C₁- bis C₆-Alkyl, vorzugsweise Methyl oder Ethyl substituiert sein kann, ferner für C₆- bis C₁₂-Arylen, welches gegebenenfalls mit weiteren Heteroatome enthaltenden aromatischen Ringen kondensiert sein kann, steht.

Bevorzugt steht X für eine Einfachbindung, C₁ bis C₅-Alkylen, C₂ bis C₅-Alkyliden, C₅ bis C₆-Cy-cloalkyliden, -O-, -SO-, -CO-, -S-, -SO₂-, oder für einen Rest der Formel (III) 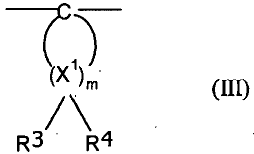 wobei
- R³ und R⁴: für jedes X¹ individuell wählbar, unabhängig voneinander Wasserstoff oder C₁ bis C₆-Alkyl, vorzugsweise Wasserstoff, Methyl oder Ethyl bedeuten und
- X¹: Kohlenstoff und
- m: eine ganze Zahl von 4 bis 7, bevorzugt 4 oder 5 bedeuten, mit der Maßgabe, dass an mindestens einem Atom X¹, R3 und R4 gleichzeitig Alkyl sind.

Für die Herstellung der erfindungsgemäß zu verwendenden Copolycarbonate wird als Diphenol abgeleitet von Formel (II) bevorzugt Bisphenol A, 2,2-Bis-(3-methyl-4-hydroxyphenyl)-propan (Dimethyl-Bisphenol A), 1,1-Bis-(4-hydroxyphenyl)-3,3,5-trimethylcyclohexan (Bisphenol TMC) sowie 1,1-Bis-(3-methyl-4-hydroxyphenyl)-3,3;5-trimethylcyclohexan (Dimethyl TMC) eingesetzt.

Für die Herstellung der erfindungsgemäß zu verwendenden Copolycarbonate geeignete Diphenole die der Struktureinheit nach Formel (II) zugrunde liegen sind beispielsweise Hydrochinon, Resorcin, Bis-(hydroxyphenyl)-alkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole, sowie deren alkylierte, kernalkylierte und kernhalogenierte Verbindungen.

Bevorzugte Diphenole sind 4,4'-Dihydroxydiphenyl, 2,2-Bis-(4-hydroxyphenyl)-1-phenyl-propan, 1,1-Bis-(4-hydroxyphenyl)-phenyl-ethan, 2,2-Bis-(4-hydroxyphenyl)propan, 2,2-Bis-(3-methyl-4-hydroxyphenyl)-propan, 2,4-Bis-(4-hydroxyphenyl)-2-methylbutan, 1,3-Bis-[2-(4-hydroxyphenyl)-2-propyl]benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol, 1,1-Bis-(4-hydroxyphenyl)-3,3,5-trimethylcyclohexan, 2,2-Bis(4-hydroxyphenyl)-1-phenyl-1H-indol-3-on, 3,3-Bis(4-hydroxyphenyl)-1-phenyl-1H-indol-2-on.

Ferner können Bisphenole der allgemeinen Formeln (IVa) und (IVb) (Isomerengemisch) (im Folgenden auch als Bisphenole der Formel (IV) bezeichnet) als weitere Diphenolverbindung verwendet werden. 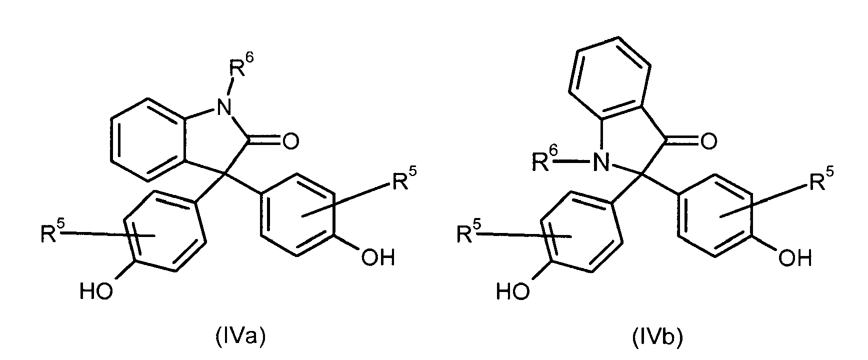 worin:
- R⁵: unabhängig voneinander für Wasserstoff oder C₁-C₁₀ Alkyl, bevorzugt für Wasserstoff oder C₁-C₆ Alkyl, besonders bevorzugt Wasserstoff oder C₁-C₄ Alkyl, ganz besonders bevorzugt für Wasserstoff oder Methyl steht
- R⁶: für C₁-C₁₀ Alkyl, bevorzugt C₁-C₆ Alkyl, besonders bevorzugt C₁-C₄ Alkyl, jeweils gegebenenfalls substituiertes Phenyl oder Benzyl, insbesondere Methyl, Phenyl oder Benzyl steht, wobei als Substituenten für Phenyl und Benzyl die bei R¹ genannten Reste bevorzugt sind.

Alkyl im Sinne der vorliegenden Erfindung ist jeweils linear oder verzweigt. 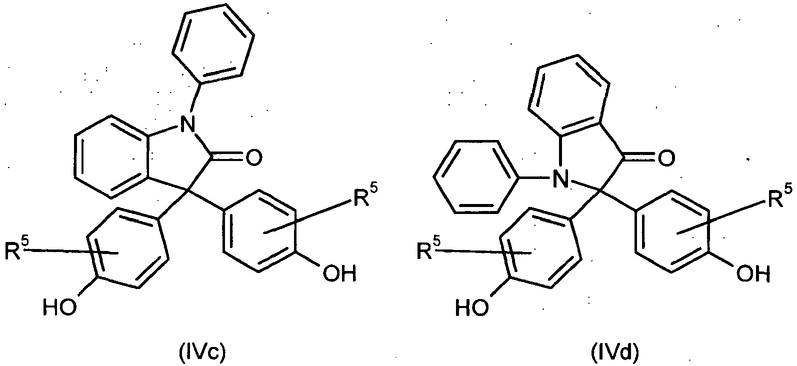 worin
R⁵ die oben genannte Bedeutung hat.

Ganz besonders bevorzugt ist das Bisphenol der Formel (IVe) und (IVf) (Isomerengemisch).

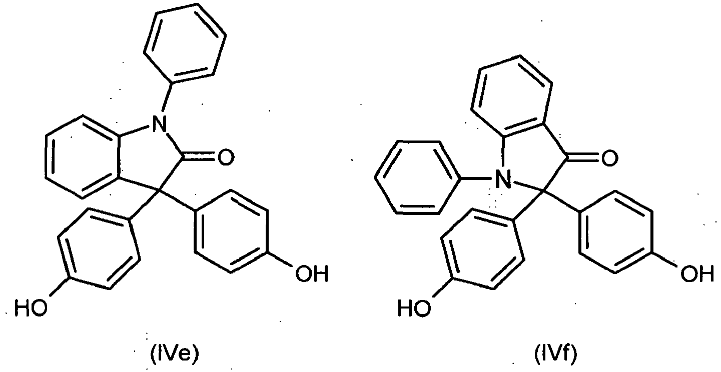

Besonders bevorzugte Diphenole sind 2,2-Bis-(4-hydroxyphenyl)-propan (Bisphenol A), und 2,2-Bis-(3-methyl-4-hydroxyphenyl)-propan (Dimethyl Bisphenol A) bzw. Bisphenol TMC sowie die Bisphenole der Formel (IV).

Ganz besonders bevorzugt sind Copolycarbonate aus dem Bisphenol (I) und Bisphenol A bzw. Bisphenol TMC bzw. Dimethyl Bisphenol A.

Diese und weitere geeignete Diphenole sind kommerziell erhältlich und z.B. in "H. Schnell, Chemistry and Physics of Polycarbonates, Interscience Publishers, New York 1964, S. 28ff; S.102ff', und in "D.G. Legrand, J.T. Bendler, Handbook of Polycarbonate Science and Technology, Marcel Dekker New York 2000, S. 72ff." beschrieben.

Das Bisphenol der Formel (I) kann sowohl allein als auch im Gemisch mit einem oder mehreren Bisphenolen der Formel (II) und / oder (IV) verwendet werden; unter Polycarbonaten im Sinne der vorliegenden Erfindung sind sowohl Homopolycarbonate als auch Copolycarbonate zu verstehen.

Copolycarbonate enthalten im Allgemeinen neben einem Diphenol ausgewählt aus Verbindungen der Formel (I) bis zu 95 Mol-%, vorzugsweise bis zu 80 Mol-%, besonders bevorzugt bis 70 Mol-% mindestens ein weiteres Diphenol, ausgewählt aus Verbindungen der Formel (II) und / oder (IV) (bezogen auf die Summe der Mole eingesetzter Diphenole). Bevorzugt enthalten die Copolycarbonate als untere Grenze mindestens 5 Mol-%, insbesondere 10 Mol-% (bezogen auf die Summe der Mole eingesetzter Diphenole) ausgewählt aus Verbindungen der Formel (II) und / oder (IV). Besonders bevorzugte Copolycarbonate enthalten 40-60, insbesondere 45-55 Mol-% Diphenol der Formel (I) und 60-40, insbesondere 45-55 Mol-% Diphenol(e) der Formel (II) und / oder (IV) (bezogen auf die Summe der Mole eingesetzter Diphenole).

Die erfindungsgemäß zu verwendenden thermoplastischen Polycarbonate und Copolycarbonate haben im Allgemeinen mittlere Molekulargewichte (Gewichtsmittel Mw, ermittelt durch Gelpermeationschromatographie GPC Messung mit Polycarbonat-Eichung) von 2 000 g/mol bis 200 000 g/mol, bevorzugt 3 000 g/mol bis 150 000 g/mol, insbesondere 5 000 g/mol bis 100 000 g/mol, ganz besonders bevorzugt 8 000 g/mol bis 80 000 g/mol, insbesondere 12 000 g/mol bis 70 000 g/mol. Molekulargewichte lassen sich auch durch die Zahlenmittel Mn angeben, welche ebenfalls nach vorheriger Eichung auf Polycarbonat mittels GPC bestimmt werden.

Als thermoplastisches Polymer wird bevorzugt wenigstens eines aus der Reihe Polycarbonat, Polyamid, Polyester, insbesondere Polybutylenterephthalat und Polyethylenterephthalat, Polylactid, Polyether, thermoplastisches Polyurethan, Polyacetal, Fluorpolymer, insbesondere Polyvinylidenfluorid, Polyethersulfone, Polyolefin, insbesondere Polyethylen und Polypropylen, Polyimid, Polyacrylat, insbesondere Poly(methyl)methacrylat, Polyphenylenoxid, Polyphenylensulfid, Polyetherketon, Polyaryletherketon, Styrolpolymerisate, insbesondere Polystyrol, Styrolcopolymere, insbesondere Styrolacrylnitrilcopolymer, Acrylnitrilbutadienstyrolblockcopolymere und Polyvinylchlorid eingesetzt.

Besonders bevorzugt sind Polycarbonate als Blend-Partner. insbesondere Styrolacrylnitrilcopolymer, Acrylnitrilbutadienstyrolblockcopolymere und Polyvinylchlorid eingesetzt.

Besonders bevorzugt sind Polycarbonate als Blend-Partner.

Die verwendeten Diphenole, wie auch alle anderen der Synthese zugesetzten Chemikalien und Hilfsstoffe, können mit den aus ihrer eigenen Synthese, Handhabung und Lagerung stammenden Verunreinigungen kontaminiert sein. Es ist jedoch wünschenswert, mit möglichst reinen Rohstoffen zu arbeiten.

Zur Gewinnung hochmolekularer Polycarbonate nach dem Phasengrenzflächenverfahren werden im Zweiphasengemisch die Alkalisalze von Diphenolen mit Phosgen umgesetzt. Das Molekulargewicht kann durch die Menge an Monophenolen, die als Kettenabbrecher wirken, wie z. B. Phenol, tert.-Butylphenol oder Cumylphenol gesteuert werden, besonders bevorzugt Phenol, tert.-Butylphenol. Bei diesen Umsetzungen entstehen praktisch ausschließlich lineare Polymere. Dies kann durch Endgruppenanalyse nachgewiesen werden. Durch gezielte Verwendung von sogenannten Verzweigern, in der Regel mehrfach hydroxylierte Verbindungen, werden dabei auch verzweigte Polycarbonate erhalten.

Die Menge an einzusetzenden Kettenabrecher beträgt 0,5 Mol-% bis 10 Mol-%, bevorzugt 1 Mol-% bis 8 Mol-%, besonders bevorzugt 2 Mol-% bis 6 Mol-% bezogen auf Mole an jeweils eingesetzten Diphenolen. Die Zugabe der Kettenabbrecher kann vor, während oder nach der Phosgenierung erfolgen, bevorzugt als Lösung in einem Lösungsmittelgemisch aus Methylenchlorid und Chlorbenzol (8-15 Gew.%-ig).

Die Polycarbonate bzw. Copolycarbonate können auch verzweigt sein. Hierzu werden bestimmte geringe Mengen, vorzugsweise Mengen zwischen 0.05 und 5 mol-%, besonders bevorzugt 0,1 bis 3 mol-%, ganz besonders bevorzugt 0,1 bis 2 mol-%, bezogen auf die Mole eingesetzter Diphenole, an trifunktionellen Verbindungen wie z. B. Isatinbiskresol (IBK) oder Phloroglucin, 4,6-Dimethyl-2,4,6-tri-(4-hydroxyphenyl)-hepten-2; 4,6-Dimethyl-2,4,6-tri-(4-hydroxyphenyl)-heptan; 1,3,5-Tri-(4-hydroxyphenyl)-benzol; 1,1,1-Tri-(4-hydroxyphenyl)-e th an (THPE); Tri-(4-hydroxyphenyl)-phenylmethan; 2,2-Bis-[4,4-bis-(4-hydroxyphenyl)-cyclohexyl]-propan; 2,4-Bis-(4-hydroxyphenyl-isopropyl)-phenol; 2,6-Bis-(2-hydroxy-5'-methyl-benzyl)-4-methylphenol; 2-(4-Hydroxyphenyl)-2-(2,4-dihydroxyphenyl)-propan; Hexa-(4-(4-hydroxyphenyl-isopropyl)-phenyl)-orthoterephthal-säureester; Tetra-(4-hydroxyphenyl)-methan; Tetra-(4-(4-hydroxyphenyl-isopropyl)-phenoxy)-methan; α, α'.α"-Tris-(4-hydroxyphenyl)-1,3,5-triisopropylbenzol;2,4-Dihydroxybenzoesäure; Trimesinsäure; Cyanurchlorid; 3,3-Bis-(3-methyl-4-hydroxyphenyl)-2-oxo-2,3-dihydroindol; 1,4-Bis-(4',4"-dihydroxytriphenyl)-methyl)-benzol

Durch den Einsatz dieser Verzweiger ergeben sich verzweigte Strukturen. Die resultierende Langkettenverzweigung führt zu rheologischen Eigenschaften der erhaltenen Polycarbonate, die sich in einer Strukturviskosität im Vergleich zu linearen Typen äußert.

Ein Verfahren zur Herstellung der erfindungsgemäß zu verwendenden Polycarbonate und Copolycarbonate ist dadurch gekennzeichnet, dass Bisphenole und evtl. Verzweiger in wässriger alkalischer Lösung gelöst werden und mit einer gegebenenfalls in einem Lösemittel gelösten Carbonatquelle wie Phosgen in einem Zweiphasengemisch aus einer wässrigen alkalischen Lösung, einem organischen Lösemittel und einem Katalysator, bevorzugt einer Aminverbindung, zur Reaktion gebracht werden. Die Reaktionsführung kann auch mehrstufig erfolgen. Solche Verfahren zur Herstellung von Polycarbonat sind als Zweiphasengrenzflächenverfahren grundsätzlich z. B. aus H. Schnell, Chemistry and Physics of Polycarbonates, Polymer Reviews, Vol. 9, Interscience Publishers, New York 1964 S. 33 ff. und aus Polymer Reviews, Vol. 10, "Condensation Polymers by Interfacial and Solution Methods", Paul W. Morgan, Interscience Publishers, New York 1965, Kap. VIII, S. 325 bekannt und die grundlegenden Bedingungen daher dem Fachmann geläufig.

Die Konzentration der Bisphenole in der wässrigen alkalischen Lösung beträgt dabei 2 bis 25 Gew.-%, bevorzugt 2 bis 20 Gew.-%, besonders bevorzugt 2 bis 18 Gew.-% und ganz besonders bevorzugt 3 bis 15 Gew.-%. Die wässrige alkalische Lösung besteht aus Wasser, in dem Hydroxide von Alkali- oder Erdalkalimetallen gelöst sind. Bevorzugt sind Natrium- und Kaliumhydroxide.

Bei der Verwendung von Phosgen als Carbonatquelle beträgt das Volumenverhältnis wässrige alkalische Lösung zu organischem Lösemittel 5:95 bis 95:5, bevorzugt 20:80 bis 80:20, besonders bevorzugt 30:70 bis 70:30 und ganz besonders bevorzugt 40:60 bis 60:40. Das Molverhältnis Bisphenol zu Phosgen ist kleiner als 1:10, bevorzugt kleiner als 1:6, besonders bevorzugt kleiner als 1:4 und ganz besonders bevorzugt kleiner als 1:3. Die Konzentration der erfindungsgemäß zu verwendenden verzweigten Polycarbonate und Copolycarbonate in der organischen Phase beträgt 1,0 bis 25 Gew.-%, bevorzugt 2 bis 20 Gew.- %, besonders bevorzugt 2 bis 18 Gew.-% und ganz besonders bevorzugt 3 bis 15 Gew.-%.

Die Konzentration der Aminverbindung beträgt bezogen auf die eingesetzte Bisphenolmenge 0,1 bis 10 mol-%, bevorzugt 0,2 bis 8 mol-%, besonders bevorzugt 0,3 bis 6 mol-% und ganz besonders bevorzugt 0,4 bis 5 mol-%.

Unter Bisphenolen sind die o.g. Diphenole, mit Anteilen der oben genannten Verzweiger, zu verstehen. Bei der Carbonatquelle handelt es sich um Phosgen, Diphosgen oder Triphosgen, bevorzugt um Phosgen. Für den Fall, dass Phosgen eingesetzt wird, kann gegebenenfalls auf ein Lösemittel verzichtet und das Phosgen direkt in das Reaktionsgemisch eingeleitet werden.

Als Katalysator können tertiäre Amine wie Triethylamin oder N-Alkylpiperidine eingesetzt werden. Als Katalysatoren geeignet sind Trialkylamine und 4-(Dimethylamino)pyridin. Besonders geeignet sind Triethylamin, Tripropylamin, Triisopropylamin, Tributylamin, Triisobutylamin, N-Methylpiperidin, N-Ethylpiperidin, und N-Propylpiperidin.

Als organisches Lösemittel kommen halogenierte Kohlenwasserstoffe wie Methylenchlorid und/oder Chlorbenzol, Dichlorbenzol, Trichlorbenzol oder Gemische davon oder aromatische Kohlenwasserstoffe, wie z. B. Toluol oder Xylole in Frage.

Die Reaktionstemperatur kann -5°C bis 100°C betragen, bevorzugt 0°C bis 80°C, besonders bevorzugt 10°C bis 70°C und ganz besonders bevorzugt 10°C bis 60°C betragen.

Alternativ können die erfindungsgemäß zu verwendenden Polycarbonate auch nach dem Schmelzumesterungsverfahren hergestellt werden. Das Schmelzumesterungsverfahren ist beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) sowie der DE-C 10 31 512 beschrieben.

Beim Schmelzumesterungsverfahren werden die bereits beim Phasengrenzflächenverfahren beschriebenen aromatischen Dihydroxyverbindungen, mit Kohlensäurediestern unter Zuhilfenahme von geeigneten Katalysatoren und gegebenenfalls weiteren Zusatzstoffen in der Schmelze umgeestert.

Kohlensäurediester im Sinne der Erfindung sind solche der Formel (6) und (7) 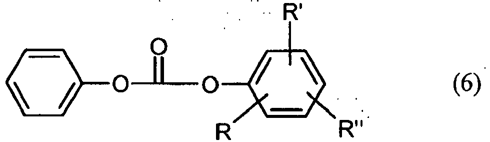 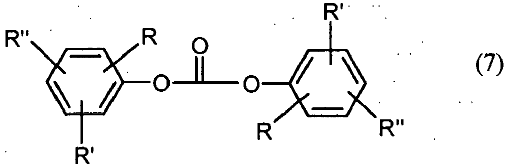 wobei 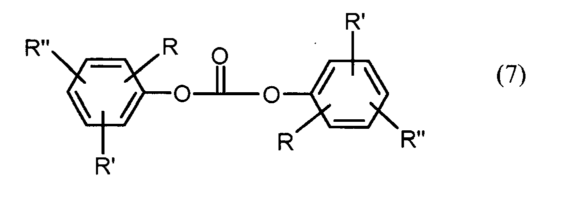 wobei
R, R' und R" unabhängig voneinander H, gegebenenfalls verzweigte C₁-C₃₄-Alkyl/Cycloakl, C₇-C₃₄-Alkaryl oder C₆-C₃₄-Aryl darstellen können,
beispielsweise
Diphenylcarbonat, Butylphenylphenylcarbonat, Di-Butylphenylcarbonat, Isobutylphenylphenylcarbonat, Di-Isobutylphenylcarbonat, tert-Butylphenyl-phenylcarbonat, Di-tert-Butylphenylcarbonat, n-Pentylphenyl-phenylcarbonat, Di-(n-Pentylphenyl)carbonat, n-Hexylphenylphenylcarbonat, Di-(n-Hexylphenyl)carbonat, Cyclohexylphenyl-p henylcarbonat, D i-Cyclohexylphenylcarbonat, Phenylphenolphenylcarbonat, Di-Phenylphenolcarbonat, Isooctylphenyl-phenylcarbonat, Di-Isooctylphenylcarbonat, n-Nonylphenyl-phenylcarbonat, Di-(n-Nonylphenyl)carbonat, Cumylphenylphenylcarbonat, Di-Cumylphenylcarbonat, Naphthylphenylphenylcarbonat, Di-Naphthylphenylcarbonat, Di-tert-Butylphenyl-phenylcarbonat, Di-(Di-tert-Butylphenyl)carbonat, Dicumylphenyl-phenylcarbonat, Di-(Dicumylphenyl)carbonat, 4-Phenoxyphenyl-phenylcarbonat, Di-(4-Phenoxyphenyl)carbonat, 3-Pentadecylphenyl-phenylcarbonat, Di-(3-Pentadecylphenyl)carbonat, Tritylphenyl-phenylcarbonat, Di-Tritylphenylcarbonat,
bevorzugt
Diphenylcarbonat, tert-Butylphenyl-phenylcarbonat, Di-tert-Butylphenylcarbonat, Phenylphenolphenylcarbonat, Di-Phenylphenolcarbonat, Cumylphenyl-phenylcarbonat, Di-Cumylphenylcarbonat,
besonders bevorzugt Diphenylcarbonat.
Es können auch Mischungen der genannten Kohlensäurediester eingesetzt werden.

Der Anteil an Kohlensäureester beträgt 100 bis 130 mol-%, bevorzugt 103 bis 120 mol-%, besonders bevorzugt 103 bis 109 mol-%, bezogen auf die Dihydroxyverbindung.

Als Katalysatoren im Sinne der Erfindung werden im Schmelzumesterungsverfahren wie in der genannten Literatur beschrieben basische Katalysatoren wie beispielsweise Alkali- und Erdalkalihydroxide und -oxide aber auch Ammonium- oder Phosphoniumsalze, im Folgenden als Oniumsalze bezeichnet, eingesetzt. Bevorzugt werden dabei Oniumsalze, besonders bevorzugt Phosphoniumsalze eingesetzt. Phosphoniumsalze im Sinne der Erfindung sind solche der Formel (IV) 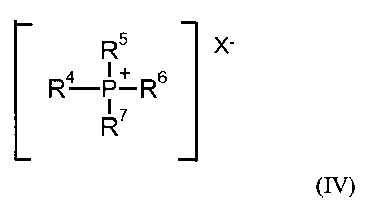 wobei
- R⁴⁻⁷: dieselben oder verschiedene C₁-C₁₀-Alkyle, C₆-C₁₀-Aryle, C₇-C₁₀-Aralkyle oder C₅-C₆-Cycloalkyle sein können, bevorzugt Methyl oder C₆-C₁₄-Aryle, besonders bevorzugt Methyl oder Phenyl, und
- X⁻: ein Anion wie Hydroxid, Sulfat, Hydrogensulfat, Hydrogencarbonat, Carbonat, ein Halogenid, bevorzugt Chlorid, oder ein Alkoholat der Formel OR sein kann, wobei R C₆-C₁₄-Aryl oder C₇-C₁₂₋Aralkyl, bevorzugt Phenyl, sein kann. Bevorzugte Katalysatoren sind

Tetraphenylphosphoniumchlorid, Tetraphenylphosphoniumhydroxid, Tetraphenylphosphoniumphenolat, besonders bevorzugt Tetraphenylphosphoniumphenolat.

Die Katalysatoren werden bevorzugt in Mengen von 10⁻⁸ bis 10⁻³ mol, bezogen auf ein mol Bisphenol, besonders bevorzugt in Mengen von 10⁻⁷ bis 10⁻⁴ mol, eingesetzt.

Weitere Katalysatoren können allein oder gegebenenfalls zusätzlich zu dem Oniumsalz verwendet werden, um die Geschwindigkeit der Polymerisation zu erhöhen. Dazu gehören Salze von Alkalimetallen und Erdalkalimetallen, wie Hydroxide, Alkoxide und Aryloxide von Lithium, Natrium und Kalium, vorzugsweise Hydroxid-, Alkoxid- oder Aryloxidsalze von Natrium. Am meisten bevorzugt sind Natriumhydroxid und Natriumphenolat. Die Mengen des Cokatalysators können im Bereich von 1 bis 200 ppb, vorzugsweise 5 bis 150 ppb und am meisten bevorzugt 10 bis 125 ppb liegen, jeweils berechnet als Natrium.

Die Umesterungsreaktion der aromatischen Dihydroxyverbindung und des Kohlensäurediester in der Schmelze wird bevorzugt in zwei Stufen durchgeführt. In der ersten Stufe findet das Aufschmelzen der aromatischen Dihydroxyverbindung und des Kohlensäurediester bei Temperaturen von 80 bis 250°C, bevorzugt 100 bis 230°C, besonders bevorzugt 120 bis 190°C unter normalem Druck in 0 bis 5 Stunden, bevorzugt 0,25 bis 3 Stunden statt. Nach Zugabe des Katalysators wird durch Anlegen von Vakuum (bis zu 2 mm Hg) und Erhöhung der Temperatur (auf bis zu 260°C) durch (ermittelt durch Messung der relativen Lösungsviskosität in Dichlormethan oder in Mischungen gleicher Gewichtsmengen Phenol/o-Dichlorbenzol geeicht durch Lichtstreuung) im Bereich von 2000 g/mol bis 18 000 g/mol, bevorzugt von 4 000 g/mol bis 15 000 g/mol.

In der zweiten Stufe wird bei der Polykondensation durch weiteres Erhöhen der Temperatur auf 250 bis 320°C, bevorzugt 270 bis 295°C, und einem Druck von <2 mm Hg das Polycarbonat hergestellt. Hierbei wird der Rest an Brüden aus dem Prozess entfernt.

Die Katalysatoren können auch in Kombination (zwei oder mehrere) miteinander eingesetzt werden.

Beim Einsatz von Alkali-/Erdalkalimetallkatalysatoren kann es vorteilhaft sein, die Alkali-/ Erdalkalimetallkatalysatoren zu einem späteren Zeitpunkt (z. B. nach der Oligocarbonatsynthese bei der Polykondensation in der zweiten Stufe) zuzusetzen.

Die Reaktion der aromatischen Dihydroxyverbindung und des Kohlensäurediester zum Polycarbonat kann diskontinuierlich oder bevorzugt kontinuierlich durchgeführt werden, beispielsweise in Rührkesseln, Dünnschichtverdampfern, Fallfilmverdampfern, Rührkesselkaskaden, Extrudern, Knetern, einfachen Scheibenreaktoren und Hochviskos-Scheibenreaktoren.

Analog des Phasengrenzflächenverfahrens können durch Einsatz mehrfunktioneller Verbindungen verzweigte Poly- oder Copolycarbonate hergestellt werden.

Bevorzugt, besonders bevorzugt oder ganz besonders bevorzugt sind Ausführungsformen, welche von den unter bevorzugt, besonders bevorzugt oder ganz besonders bevorzugt bzw., vorzugsweise etc. genannten Parametern, Verbindungen, Definitionen und Erläuterungen Gebrauch machen.

Die in der Beschreibung aufgeführten allgemeinen oder in Vorzugsbereichen aufgeführten Definitionen, Parameter, Verbindungen und Erläuterungen können jedoch auch untereinander, also zwischen den jeweiligen Bereichen und Vorzugsbereichen beliebig kombiniert werden.

Die erfindungsgemäß zu verwendenden Polycarbonate und Copolycarbonate können in bekannter Weise aufgearbeitet und zu beliebigen Formkörpern verarbeitet werden, beispielsweise durch Extrusion, Spritzguss oder Extrusionsblasformen.

Den erfindungsgemäß zu verwendenden Polycarbonaten und Copolycarbonaten können noch andere aromatische Polycarbonate und/oder andere aromatische Polyestercarbonate und/oder andere aromatische Polyester in bekannter Weise zugemischt werden, beispielsweise durch Compoundierung.

Den erfindungsgemäßen Polycarbonaten und Copolycarbonaten können noch andere aromatische Polycarbonate und/oder andere aromatische Polyestercarbonate und/oder andere aromatische Polyester in bekannter Weise zugemischt werden, beispielsweise durch Compoundierung.

Die Zusammensetzung kann weitere handelsübliche Polymeradditive wie Flammschutzmittel, Flammschutzsynergisten, Antidrippingmittel (beispielsweise Verbindungen der Substanzklassen der fluorierten Polyolefine, der Silikone sowie Aramidfasern), Gleit- und Entformungsmittel (beispielsweise Pentaerythrittetrastearat), Nukleiermittel, Stabilisatoren, Antistatika (beispielsweise Leitruße, Carbonfasern, Carbon Nanotubes sowie organische Antistatika wie Polyalkylenether, Alkylsulfonate oder Polyamid-haltige Polymere), sowie Farbstoffe und Pigmente in solchen Mengen enthalten, die die mechanischen Eigenschaften der Zusammensetzung nicht insoweit schädigen, dass das Zieleigenschaftsprofil (kein splitterndes Bruchversagen bei -10°C) nicht mehr erfüllt wird.

Als Flammschutzmittel werden vorzugsweise Sulfonsäuresalze wie Kaliumperfluorbutansulfonsäure ooder Kaliumdiphenylsulfonsulfonat, bromierte Oligobisphenole, PTFE oder PTFE-Compounds sowie phosphorhaltige Flammschutzmittel eingesetzt, insbesondere ausgewählt aus den Gruppen der mono- und oligomeren Phosphor- und Phosphonsäureester, Phosphonatamine und Phosphazene, wobei auch Mischungen von mehreren Komponenten ausgewählt aus einer oder verschiedener dieser Gruppen als Flammschutzmittel zum Einsatz kommen können. Auch andere hier nicht speziell erwähnte, vorzugsweise halogenfreie Phosphorverbindungen können alleine oder in beliebiger Kombination mit anderen, vorzugsweise halogenfreien Phosphorverbindungen eingesetzt werden. Beispielsweise sind als Phosphorverbindungen geeignet: Tributylphosphat, Triphenylphosphat, Trikresylphosphat, Diphenylkresylphosphat, Diphenyloctylphosphat, Diphenyl-2-ethylkresylphosphat, Tri-(isopropylphenyl)-phosphat, Resorcin-verbrücktes Di- bzw. Oligophosphat und Bisphenol A verbrücktes Di- bzw. Oligophosphat. Der Einsatz von oligomeren Phosphorsäureestern, die sich vom Bisphenol A ableiten, ist insbesondere bevorzugt. Als Flammschutzmittel geeignete Phosphorverbindungen sind bekannt (vgl. z.B. EP-A 0 363 608, EP-A 0 640 655) oder lassen sich nach bekannten Methoden in analoger Weise herstellen (z.B. Ullmanns Enzyklopädie der technischen Chemie, Bd. 18, S. 301 ff. 1979; Houben-Weyl, Methoden der organischen Chemie, Bd. 12/1, S. 43; Beilstein Bd. 6, S. 177).

Der Zusatz von Additiven dient der Verlängerung der Nutzungsdauer oder der Farbe (Stabilisatoren), der Vereinfachung der Verarbeitung (z.B. Entformer, Fließhilfsmittel, Antistatika) oder der Anpassung der Polymereigenschaften an bestimmte Belastungen (Schlagzähmodifikatoren, wie Kautschuke; Flammschutzmittel, Farbmittel, Glasfasern).

Diese Additive können einzeln oder in beliebigen Mischungen oder mehreren verschiedenen Mischungen der Polymerschmelze zugesetzt werden und zwar direkt bei der Isolierung des Polymeren oder aber nach Aufschmelzung von Granulat in einem sogenannten Compoundierungsschritt. Dabei können die Additive beziehungsweise deren Mischungen als Feststoff, also als Pulver, oder als Schmelze der Polymerschmelze zugesetzt werden. Eine andere Art der Dosierung ist die Verwendung von Masterbatches oder Mischungen von Masterbatches der Additive oder Additivmischungen.

Geeignete Additive sind beispielsweise beschrieben in "Additives for Plastics Handbook, John Murphy, Elsevier, Oxford 1999", im "Plastics Additives Handbook, Hans Zweifel, Hanser, München 2001" oder in WO 99/55772, S. 15-25.

Als Thermostabilisator eignen sich bevorzugt Tris-(2,4-di-tert-butylphenyl)phosphit (Irgafos 168), Tetrakis(2,4-di-tert.-butylphenyl)[1,1-biphenyl]-4,4'-diylbisphosphonit, Triisoctylphosphat, Octadecyl-3-(3,5-di-tert-butyl-4-hydroxyphenyl)propionat (Irganox 1076 von BASF), Bis(2,4-dicumyl-phenyl)pentaerythritoldiphosphit (Doverphos S-9228-PC), Bis(2,6-di-tert.-butyl-4-methylphenyl)-pentaerythritoldiphosphit (ADK STAB PEP-36) oder Triphenylphosphin. Sie werden allein oder im Gemisch (z. B. Irganox B900 von BASF oder Doverphos S-9228-PC mit Irganox B900 bzw. Irganox 1076) eingesetzt.

Als UV-Stabilisatoren sind organische UV-Stabilisatoren geeignet. Die UV-Stabilisatoren sind bevorzugt ausgewählt aus der Gruppe, die Benzotriazole (z. B. Tinuvine der Fa. Ciba), Triazine CGX-06 der Fa. Ciba), Benzophenone (Uvinule der Fa. BASF), Cyanacrylate (Uvinule der Fa. BASF), Zimtsäureester und Oxalanilide sowie Mischungen dieser UV-Stabilisatoren umfaßt.

Beispiele für geeignete UV-Absorber sind:
a) Malonester der Formel (I): 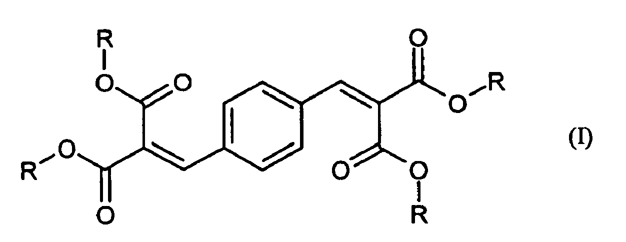 Worin R Alkyl bedeutet. Bevorzugt steht R für C1-C6-Alkyl, insbesondere für C1-C4-Alkyl und besonders bevorzugt für Ethyl.
b) Benzotriazol-Derivate nach Formel (II): 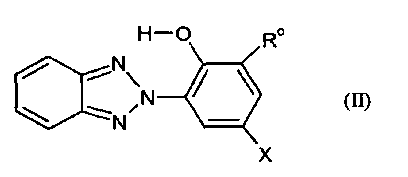 In Formel (II) sind R° und X gleich oder verschieden und bedeuten H oder Alkyl oder Alkylaryl.
   Bevorzugt sind dabei Tinuvin® 329 mit X = 1,1,3,3-Tetramethylbutyl und R° = H, Tinuvin® 350 mit X = tert-Butyl und R⁰ = 2-Butyl und Tinuvin® 234 mit X und R° = 1,1-Dimethyl-1-phenyl
c) Dimere Benzotriazol-Derivate nach Formel (III): 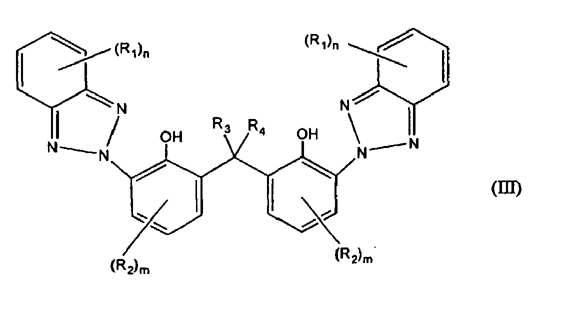 In Formel (III) sind R1 und R2 gleich oder verschieden und bedeuten H, Halogen, C1- C10-Alkyl, C5- C10-Cycloalkyl, C7-C13 -Aralkyl, C6-C14 -Aryl, -OR5 oder -(CO)-O-R 5 mit R5 = H oder C1-C4-Alkyl.
   In Formel (III) sind R3 und R4 ebenfalls gleich oder verschieden und bedeuten H, C1-C 4-Alkyl, C5-C6 -Cycloalkyl, Benzyl oder C6-C14-Aryl.
   In Formel (III) bedeuten m 1,2 oder 3 und n 1,2,3 oder 4.
   Bevorzugt ist dabei Tinuvin® 360 mit R1 = R3 = R4 = H; n = 4; R2 = 1,1,3,3-Tetramethylbutyl;
   m = 1
d) Dimere Benzotriazol-Derivate nach Formel (IV): 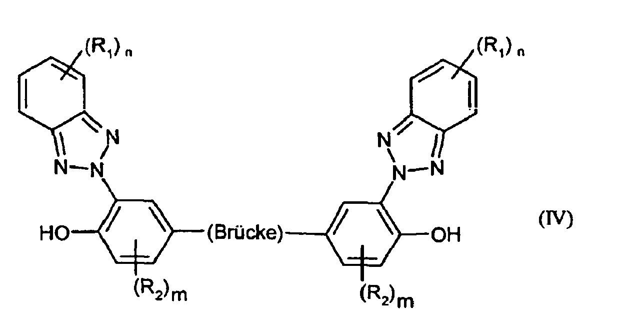 worin die Brücke 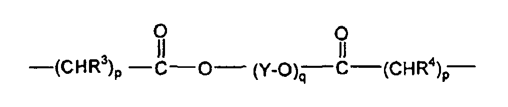 bedeutet, R₁ , R₂, m und n die für Formel (III) genannte Bedeutung haben, und worin p eine ganze Zahl von 0 bis 3 ist, q eine ganze Zahl von 1 bis 10 ist, Y gleich -CH2-CH2-, -(CH2)3-, -(CH2)4-, - (CH2)5-, -(CH2)6-, oder CH(CH3)-CH2-ist und R3 und R4 die für Formel (III) genannte Bedeutung haben.
   Bevorzugt sind dabei Tinuvin® 840 mit R1 = H; n = 4; R2 = tert-Butyl; m = 1; R2 ist in ortho-Stellung zur OH-Gruppe angebracht; R3 = R4 = H; p = 2; Y = -(CH2)5-; q = 1
e) Triazin-Derivate nach Formel (V): 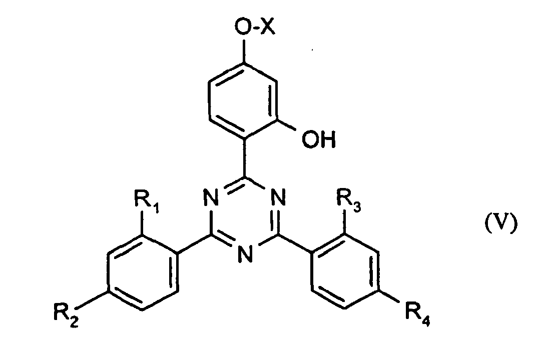 worin R1, R2, R3, R4 gleich oder verschieden sind und H, Alkyl, Aryl, CN oder Halogen sind und X gleich Alkyl, vorzugsweise iso-Octyl, ist.
   Bevorzugt ist dabei Tinuvin® 1577 mit R1 = R2 = R3 = R4 = H; X = Hexyl sowie Cyasorb® UV-1 164 mit R1 = R2 = R3 = R4 = Methyl; X Octyl
f) Triazin-Derivate der folgenden Formel (Va): 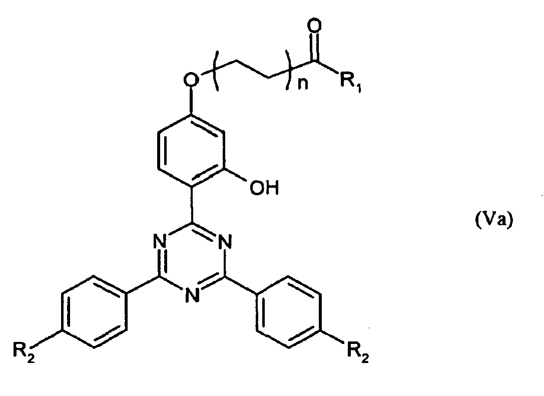 worin R1 gleich C1 Alkyl bis C17-Alkyl bedeutet, R2 gleich H oder C1-Alkyl bis C4-Alkyl bedeutet und n gleich 0 bis 20 ist.
g) Dimere Triazin-Derivate der Formel (VI):

### h) Diarylcyanoacrylate der Formel (VII):

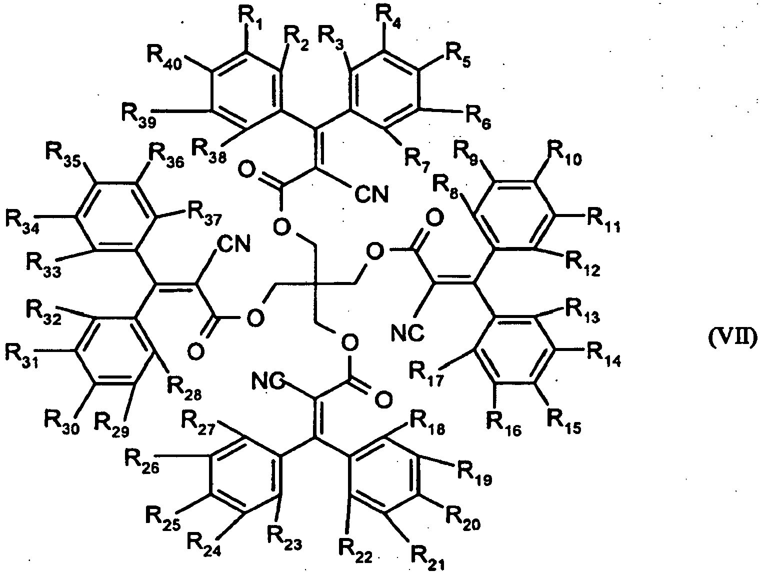

worin R bis R40 gleich oder verschieden sein können und H, Alkyl, CN oder Halogen bedeuten.

Bevorzugt ist dabei Uvinul® 3030 mit R1 bis R40 = H.

Besonders bevorzugte UV-Stabilisatoren für die erfindungsgemäß zu verwendenden Formmassen sind Verbindungen aus der Gruppe, die aus den Benzotriazolen (b) und dimeren Benzotriazolen (c und d), den Malonestern (a) und den Cyanacrylaten (h) sowie Mischungen dieser Verbindungen besteht.

Die UV-Stabilisatoren werden eingesetzt in Mengen von 0,01 Gew.-% bis 15,00 Gew.-% bezogen auf die Formmasse, bevorzugt in Mengen von 0,05 Gew.-% bis 1,00 Gew.-%, besonders bevorzugt in Mengen von 0,08 Gew.-% bis 0,5 Gew.-% und ganz besonders bevorzugt in Mengen von 0,1 Gew.-% bis 0,4 Gew.-% bezogen auf die Gesamtzusammensetzung.

Die den erfindungsgemäß zu verwendenden Zusammensetzungen gegebenenfalls zugesetzten Entformungsmittel sind bevorzugt ausgewählt aus der Gruppe, die Pentäerythrittetrastearat, Glycerinmonostearat, Stearylstearat und Propoandiolstearat sowie deren Mischungen umfaßt. Die Entformungsmittel werden eingesetzt in Mengen von 0,05 Gew.-% bis 2,00 Gew.-% bezogen auf die Formmasse, bevorzugt in Mengen von 0,1 Gew.-% bis 1,0 Gew.-%, besonders bevorzugt in Mengen von 0,15 Gew.-% bis 0,60 Gew.-% und ganz besonders bevorzugt in Mengen von 0,2 Gew.-% bis 0,5 Gew.-% bezogen auf die Gesamtzusammensetzung.

Des weiteren können Farbmittel, wie organische Farbstoffe oder Pigmente oder anorganische Pigmente, Ruß, IR-Absorber, einzeln, im Gemisch oder auch in Kombination mit Stabilisatoren, Glasfasern, Glas(hohl)kugeln, anorganischen Füllstoffen oder organische oder anorganische Streupigmente zugesetzt werden.

Die Formmassen können zur Herstellung von Formkörpern jeder Art verwendet werden. Diese können zum Beispiel durch Spritzguss, Extrusion und Blasformverfahren hergestellt werden. Eine weitere Form der Verarbeitung ist die Herstellung von Formkörpern durch Tiefziehen aus zuvor hergestellten Platten oder Folien.

Die erfindungsgemäß zu verwendenden (Co)polycarbonate und (Co)polycarbonatZusammensetzungen können auf üblichen Maschinen, beispielsweise auf Extrudern oder Spritzgussmaschinen, zu beliebigen Formkörpern bzw. Formteilen, zu Folien und Folienlaminate oder Platten oder Flaschen, in üblicher Weise verarbeitet werden.

Die Compoundierung der einzelnen Bestandteile kann in bekannter Weise sowohl sukzessive als auch simultan erfolgen.

Die so erhältlichen (Co)polycarbonate können zur Herstellung von Extrudaten (Platten, Folien und deren Laminate; z. B. für Kartenanwendungen oder Foliensubstrate für Displays wie LCDs und Rohre) und Formkörpern (Flaschen), insbesondere solchen zur Verwendung im transparenten Bereich, besonders im Bereich optischer Anwendungen wie z.B. Platten, Stegplatten, Verglasungen, Streu- oder Abdeckscheiben, Lampenabdeckungen, Kunststoffabdeckscheiben, Lichtleitelemente sowie LED-Anwendungen verwendet werden. Weiterhin können sie zur Herstellung von Gegenständen für den E/E und IT-Bereich verwendet werden.

In einer besonderen Ausführungsform ist die weitere Schicht des erfindungsgemäßen mehrschichtigen Erzeugnisses eine Metallschicht, auf die vorzugsweise noch eine Schutzschicht, beispielsweise zum Korrosionsschutz aufgetragen wird. Die korrosionsmindernde Schutzschicht kann in einem PECVD (*plasma enhanced chemical vapour deposition*) oder Plasmapolymerisationsprozess aufgebracht werden. Hierbei werden niedrigsiedende Precursoren haupsächlich auf Siloxan Basis in ein Plasma verdampft und dadurch aktiviert, so dass sie einen Film bilden können. Typsiche Substanzen hierbei sind Hexamethyldisiloxan (HMDSO), Hexamethyldisilazan (HMDS), Tetrametyldisiloxan, Decamethylcyclopentasiloxan, Octamethylcyclotetrasiloxan und Trimethoximethylsilan. Besonders bevorzugt ist HMDSO.

In einer weiteren besonderen Ausführungsform kann das Substrat vor der Metallisierung einer geeigneten Vorbehandlung unterzogen werden, wie z. B. eine Plasmavorbehandlung, mit dem Ziel, die Substratoberfläche zu aktivieren oder zu reinigen.

Die (Co)polycarbonat-Zusammensetzungen können zur Herstellung von Compounds, Blends und Bauteilen verwendet werden, bei denen optische, thermische und mechanische Eigenschaften geputzt werden, wie beispielsweise Gehäuse, Gegenstände im E/E-Bereich, wie Stecker, Schalter, Platten, Lampenhalterungen, -abdeckungen, Automobilbereich wie Lampenfassungen und -abdeckungen, Verscheibungen, Linsen, Kollimatoren, Leuchtdioden oder diffusor sheets für Displays und andere Anwendungen.

Die erfindungsgemäß zu verwendenden Polycarbonate und Copolycarbonate, gegebenenfalls in Abmischung mit anderen Thermoplasten wie beispielsweise Pfropfpolymerisate auf Basis Acrylnitril/-Butadien/Styrol oder auf Acrylatkautschuk basierende Pfropfcopolymerisate (siehe beispielsweise die in EP-A 640 655 beschriebenen Pfropfpolymerisate) und/oder üblichen Additiven, können zu beliebigen Formkörpern/Extrudaten verarbeitet überall dort eingesetzt werden, wo bereits bekannte Polycarbonate, Polyestercarbonate und Polyester eingesetzt werden. Weitere mögliche Anwendungen der Polycarbonate sind:
1. Sicherheitsscheiben, die bekanntlich in vielen Bereichen von Gebäuden, Fahrzeugen und Flugzeugen erforderlich sind, sowie als Schilde von Helmen.
2. Herstellung von Folien und Folienlaminaten.
3. Automobilscheinwerfer, bezels, Blinker, Reflektoren
4. Als transluzente Kunststoffe mit einem Gehalt an Glasfasern für lichttechnische Zwecke, als transluzente Kunststoffe mit einem Gehalt an Bariumsulfat, Titandioxid und/oder Zirkoniumoxid
5. Zur Herstellung von Präzisionsspritzgussteilen, wie beispielsweise Linsen, Kollimatoren, Linsenhalterungen, Lichtleitelementen und LED-Anwendungen.
6. Als Elektroisolierstoffe für elektrische Leiter und für Steckergehäuse sowie Steckverbinder
7. Gehäuse für elektrische Geräte
8. Schutzbrillen, Visiere
9. Extrudierte Formkörper wie Platten und Folien
10. LED-Anwendungen (Sockel, Reflektoren, heat sinks),

Die folgenden Beispiele dienen der weiteren Erläuterung der Erfindung.

### Beispiele

Die Copolycarbonate wurden mittels Vicat-Temperatur und der relativen Lösungsviskosität ηᵣₑₗ charakterisiert. Weiterhin wurden die Copolycarbonate bezüglich deren thermischer Längenausdehnungskoeffizienten untersucht.

### Ermittlung der Vicat-Temperatur und der relativen Lösungsviskosität

Die Vicat-Temperatur VST B50 wird nach DIN ISO 306, Methode B bei einer Heizlast von 50 K/h ermittelt. Die relative Lösungsviskosität wurde in Methylenchlorid als Lösungsmittel bei einer Konzentration von 0,5 g/l und bei einer Temperatur von 25°C mit einem Ubbelohde-Viskosimeter bestimmt (DIN 51562).

### Ermittlung der thermischen Längenausdehnungskoeffizienten:

Die Längenausdehnungskoeffizienten werden mittels eines Mettler TMA 841-Messgerätes unter Stickstoff ermittelt (Meßbereich 23 - 55°C). Als Norm kommt die ASTM E 831 zur Anwendung.
Die für die Messung benötigten Probekörper (Flachstab 80 x 10 x 4 mm) werden nach Trockung des Granulates bei 130 °C über Nacht durch Spritzguß hergestellt. Gemessen wird jeweils quer und
längs des Probekörpers.

### Beispiel 1

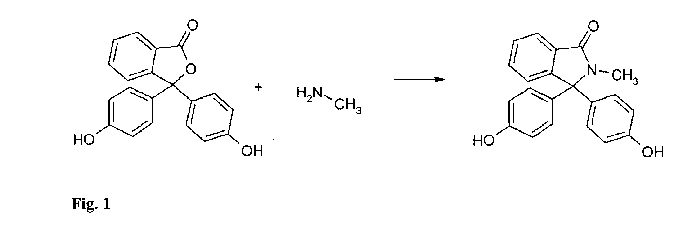

Die Synthese des erfindungsgemäßen Monomerbausteins erfolgt nach Fig. 1.

In einem Planschliffreaktor wird eine Lösung aus 2 kg (20,2 mol) N-Methylpyrrolidon (NMP) und 1273,3 g (4 mol) Phenolphtalein vorgelegt. Unter Rühren werden 2 Liter Wasser und anschließend 18 mol einer 40 %-igen wässrigen Methylaminlösung zugegeben. Bei Zugabe des Methylamins verfärbt sich die Reaktionslösung violett. Unter Nutzung eines Trockeneiskühlers wird danach für 8 Stunden bei 82 °C nachgerührt. Die Färbung des Reaktionsansatzes verändert sich dabei dunkelgelblich. Nach Reaktionsende fällt man den Reaktionsansatz mittels eines Tropftrichters unter Rühren in eine salzsaure Wasservorlage.

Das weiß ausgefallene Reaktionsprodukt wird mit 2 Liter Wasser aufgeschlämt und anschließend mittels einer G3-Fritte abgesaugt. Das erhaltene Rohprodukt wird in 3,5 Liter einer verdünnten Natronlauge (16 mol) wieder gelöst und wiederum in einer salzsauren Wasservorlage ausgefällt. Das umgefällte Rohprodukt wird mehrmals mit 2 Liter Wasser aufgeschlämt und danach jeweils abgesaugt. Diese Waschprozedur wird so lange wiederholt, bis die Leitfähigkeit des Waschwassers kleiner 15 µS ist.

Das so erhaltene Produkt wird bei 90 °C im Vakuumtrockenschrank bis zur Massenkonstanz getrocknet.

Man erhielt nach 4-maliger Experimentdurchführung jeweils folgende Ausbeuten:
1a) 950 g eines weißen Feststoffes
1b) 890 g eines weißen Feststoffes
1c) 1120 g eines weißen Feststoffes
1d) 1050 g eines weißen Feststoffes

Die Schmelzpunktbestimmung des Produktes ergab 264 °C.

Die Charakterisierung des erhaltenen Bisphenols erfolgte mittels ¹H-NMR-Spektroskopie.

### Beispiel 2

### Synthese des erfindungsgemäßen Copolycarbonates:

Zu einer mit Stickstoff inertisierten Lösung von 532,01 g (1,6055 mol) Bisphenol A (BPA), 2601,36 g (11,39 mol) Bisphenol aus Beispiel 1, 93,74 g (0,624 mol, 4,8 mol-% bzgl. Diphenole) p-tert.-Butylphenol (BUP) als Kettenabbrecher und 1196 g (29,9 mol) Natriumhydroxid in 25,9 Liter Wasser werden 11,79 Liter Methylenchlorid und 14,1 Liter Chlorbenzol hinzugegeben. Bei einem pH-Wert von 12,5 - 13,5 und 20 °C leitet man 2,057 kg (20,8 mol) Phosgen ein. Um den pH-Wert nicht unter 12,5 fallen zu lassen, wurde während der Phosgenierung 30 %-ige Natronlauge zugegeben. Nach beendeter Phosgenierung und Spülung mit Stickstoff rührt man für weitere 30 Minuten und gibt dann 14,7 g (0,13 mol, 1 mol-% bzgl. Diphenole) N-Ethylpiperidin als Katalysator zu und lässt 1 Stunde nachrühren. Die organische Phase wird nach Abtrennen der wässrigen Phase und Ansäuern mit Phosphorsäure mittels eines Separators mehrmals mit Wasser salzfrei gewaschen.
Die organische Phase wird abgetrennt und einem Lösungsmittelaustausch Chlorbenzol gegen Methylenchlorid unterworfen. Die aufkonzentrierte Copolycarbonatlösung in Chlorbenzol wird anschließend mit Hilfe eines Ausdampfextruders vom Lösungsmittel befreit. Die erhaltenen Polycarbonatschmelzestränge werden in einem Wasserbad gekühlt, abgezogen und abschließend granuliert.

Man erhält transparentes Polycarbonatgranulat (Analytik siehe Tabelle 1).

### Beispiele 3 bis 5:

### Weitere Synthesebeispiele zu erfindungsgemäßen Copolycarbonaten:

Die Copolycarbonate der Beispiele 3 bis 5 wurden analog Beispiel 2 hergestellt (Ergebnisse s. Tabelle 1).

**Tabelle 1:**

| Copolycarbonat aus | Beispiel 5 | Beispiel 4 | Beispiel 3 | Beispiel 2 |
|---|---|---|---|---|
| Bisphenol aus Beispiel 1 | | | | |
| [mol-%] | 55,5 | 25,0 | 18,0 | 12,4 |
| [Gew.-%] | 62,2 | 32,6 | 24,2 | 17,0 |
| Bisphenol A (BPA) | | | | |
| [mol-%] | 44,5 | 75 | 82 | 87,6 |
| [Gew.-%] | 37,8 | 67,4 | 75,8 | 83 |
| Vicat-temperatur [°C] | 228,2 | 188,3 | 177,4 | 168,6 |
| Glastemperatur Tg [°C] | 230,0 | 189,9 | 178,9 | 168,7 |
| ηᵣₑₗ | 1,235 | 1,240 | 1,237 | 1,238 |

Als Vergleichsproben werden Apec®-Proben (Bayer MaterialScience AG, Deutschland) mit den in Tabelle 2 angegebenen Vicat-Temperaturen verwendet. Diese stellen den nächstgelegenen Stand der Technik für Polycarbonate bei Hochtemperaturanwendungen dar.

**Tabelle 2:**

| Vergleichsbeispiele | 6 | 7 | 8 | 9 |
|---|---|---|---|---|
| Vicat-Temperatur [°C] | 158 | 173 | 183 | 203 |
| Glastemperatur Tg [°C] | 157,2 | 169,9 | 179,3 | 204 |
| Ausdehnungskoeffzient quer [10⁻⁶/K] | 68,0 | 68,0 | 66,0 | 66,0 |
| Ausdehnungskoeffzient längs [10⁻⁶/K] | 67,0 | 67,0 | 67,0 | 64,0 |
| Erfindungsgemäße Probe aus Beispiel | 2 | 3 | 4 | 5 |
| Vicat-Temperatur [°C] | 168,6 | 177,4 | 188,3 | 228,2 |
| Ausdehnungskoeffzient quer [10⁻⁶/K] | 65,0 | 63,4 | 63,4 | 59,7 |
| Ausdehnungskoeffzient längs [10⁻⁶/K] | 63,1 | 64,0 | 62,8 | 59,4 |

Im Vergleich mit den Vergleichsproben 6 - 9 stellt man fest, dass die erfindungsgemäßen Copolycarbonate aus den Beispielen 2 - 5 bei vergleichbaren Vicat-Temperaturen signifikant niedrigere Werte für den thermischen Ausdehnungskoeffizienten aufweisen. Dies war für den Fachmann nicht zu erwarten. Insbesondere das Copolycarbonat aus Beispiel 5 zeigt mit einer Vicat-Temperatur von 228,2 °C die geforderten Anforderungen an Reflektormaterialien bezüglich thermischer Stabilität und reduziertem Ausdehnungskoeffizienten.

### Thermische und mechanische Eigenschaften :

Die rheologischen Eigenschaften wurden über die Schmelze-Volumenfließrate (MVR) nach ISO 1133 bestimmt.

Die mechanischen Eigenschaften wurden über Zug-Modul, Streckspannung, Streckdehnung und nominelle Bruchdehnung nach ISO 527-1, -2, Charpy-Schlagzähigkeit nach ISO 179-1 eU und Charpy-Kerbschlagzähigkeit nach ISO 179-1 eA bestimmt.

Die thermischen Eigenschaften wurden über Formbeständigkeitstemperatur HDT (A und B) nach ISO 75-1, -2 und über die Vicat-Erweichungstemperatur nach ISO 306 ermittelt.

In der Tabelle 3 ist eine Gesamtübersicht über alle thermischen und mechanischen Eigenschaften der erfindungsgemäßen Copolycarbonate im Vergleich zu Vergleichsproben mit ähnlicher Vicat-Temperatur aufgeführt. Man kann erkennen, dass die mechanischen Eigenschaften der erfindungsgemäßen Materialien aus Beispiel 2 - 5 keine Verschlechterung gegenüber den erfindungsgemäßen Vergleichsproben 1 - 4 aufweisen. Wie oben in Tabelle 2 gezeigt, weisen sie allerdings den Vorteil von kleineren linearen thermischen Ausdehungskoeffizieneten auf.

Außerdem zeigen die erfindungsgemäßen Proben ein besseres Fließverhalten im Vergleich zu nicht erfindungsgemäßen Proben mit gleicher Vicat-Temperatur (siehe Beispiele 2 und 6 bzw. 3 und 7).

**Tabelle 3**

| | | | Vergleichsproben | | | | Erfindungsgemäße Proben | | | |
|---|---|---|---|---|---|---|---|---|---|---|
| | | | 6 | 7 | 8 | 9 | 2 | 3 | 4 | 5 |
| Mn | | | 10150 | 10544 | 9977 | 11456 | 9092 | 10004 | 9629 | 10328 |
| Mw | | | 25370 | 26253 | 26424 | 29061 | 22291 | 23228 | 24153 | 24257 |
| D | | | 2,50 | 2,49 | 2,65 | 2,59 | 2,45 | 2,32 | 2,51 | 2,35 |
| Eta rel | | | 1,257 | 1,253 | 1,249 | 1,248 | 1,238 | 1,238 | 1,241 | 1,235 |
| 1. Messung - 2. Aufheizen | | °C | 156,7 | 169,9 | 180,8 | 204 | 168,6 | 178,5 | 190,1 | 230,1 |
| 2. Messung - 2. Aufheizen | | °C | 157,7 | 169,9 | 177,8 | 204 | 168,8 | 179,2 | 189,6 | 229,9 |
| Durchschnitts-Temperatur | | °C | **157,2** | **169,9** | **179,3** | **204,0** | **168,7** | **178,9** | **189,9** | **230,0** |
| Schmelze-Volumenfließrate (MVR) (330 °C; 2,16 kg) | | cm³/ (10 min) | 45 | 24 | 18 | 8 | 46,0 | 31,0 | 17,2 | 2,2 |
| Zug-Modul (1 mm/min) | | MPa | 2400 | 2400 | 2400 | 2400 | 2407 | 2487 | 2532 | 2774 |
| Streckspannung (50 mm/min) | | MPa | 68 | 72 | 74 | 76 | 70,6 | 73,8 | 77,7 | 92,5 |
| Streckdehnung (50 mm/min) | | % | 6,2 | 6,4 | 6,8 | 6,9 | 6,6 | 6,8 | 7,1 | 7,7 |
| Nominelle Bruchdehnung (50mm/min) | | % | 96 | 89 | 89 | 57 | 78 | 77 | 61 | 15 |
| | Z | kJ/m² | - | - | - | - | - | - | - | - |
| Charpy-Schlagzähigkeit (23°C) | S | | - | - | - | - | - | - | - | - |
| | N | | 10 x NB | 10 x NB | 10 x NB | 10 x NB | 10 x NB | 10 x NB | 10 x NB | 10 x NB |
| | Z | kJ/m² | - | - | - | - | - | - | - | - |
| Charpy-Schlagzähigkeit (-20°C) | S | | - | - | - | - | - | - | - | - |
| | N | | 10 x NB | 10 x NB | 10 x NB | 10 x NB | 10 x NB | 10 x NB | 10 x NB | 10 x NB |
| Charpy-Kerbschlagzähigkeit (23 °C; 3,0 mm) | | kJ/m² | 10 x 13b | 10 x 12b | 10 x 10b | 10 x 8b | 10 x 16b | 10 x 17b | 10 x 16b | 10 x 17b |
| Formbeständigkeitstemperatur HDT, Af (1,80 MPa) | | °C | 138 | 149 | 158 | 173 | 144,9 | 154,1 | 164,1 | 201,6 |
| Formbeständigkeitstemperatur HDT, Bf (0,45 MPa) | | °C | 150 | 163 | 173 | 192 | 158,9 | 167,8 | 178,6 | 218,1 |
| Vicat-Erweichungstemperatur (50 N, 120 K/h) | | °C | **158** | **173** | **183** | **203** | **168,6** | **177,4** | **188,3** | **228,2** |

| | | | | | | | | | | |
|---|---|---|---|---|---|---|---|---|---|---|
| Z: zäh S: spröde N: nicht gebrochen NB: not broken (nicht gebrochen) b: brittle (spröde) | | | | | | | | | | |

## Patentansprüche

1. Mehrschichtiges Erzeugnis enthaltend ein Substrat, das mindestens auf einer Seite eine weitere Schicht aufweist, wobei das Substrat aus wenigstens einem (Co)Polycarbonat oder wenigstens einem Blend enthaltend ein oder mehrere (Co)Polycarbonate und ein oder mehrere thermoplastische Polymere hergestellt wird, **dadurch gekennzeichnet, dass** die (Co)Polycarbonat(e) Bisphenole der Formel (Ia) als wiederkehrende Monomereinheit im Polymer 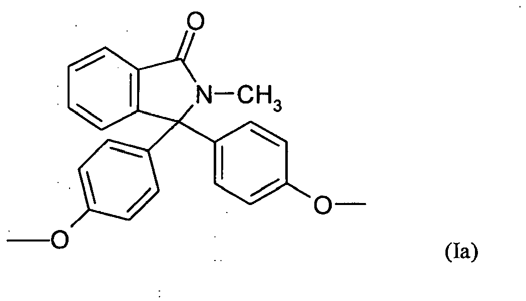 enthalten.

2. Mehrschichtiges Erzeugnis gemäß Anspruch 1, **dadurch gekennzeichnet, dass** die (Co)Polycarbonat(e) bis zu 95 Mol-% (bezogen auf die Menge eingesetzter Diphenole) eines oder mehrerer Diphenole enthalten, ausgewählt aus Diphenolen der Formel (II) und Diphenolen der Formel (IV), wobei Diphenole der Formel (IV) ein Isomerengemisch aus Diphenolen der Formeln (IVa) und (IVb) sein können 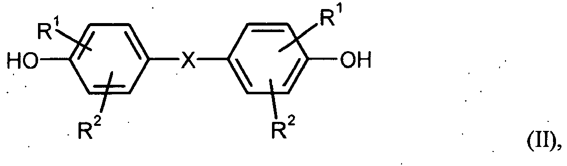 in welcher
R¹ und R² unabhängig voneinander für Wasserstoff, C₁-C₁₈-Alkyl-, C₁-C₁₈-Alkoxy, Halogen, jeweils gegebenenfalls substituiertes Aryl oder Aralkyl, stehen, und
X für eine Einfachbindung, -SO₂- -CO-, -O-, -S-, C₁- bis C₆-Alkylen, C₂- bis C₅-Alkyliden oder C₅- bis C₆- Cycloalkyliden, welches mit C₁- bis C₆-Alkyl substituiert sein kann, für C₆-C₁₂-Arylen, welches gegebenenfalls mit weiteren Heteroatome enthaltenden aromatischen Ringen kondensiert sein kann, steht, und / oder 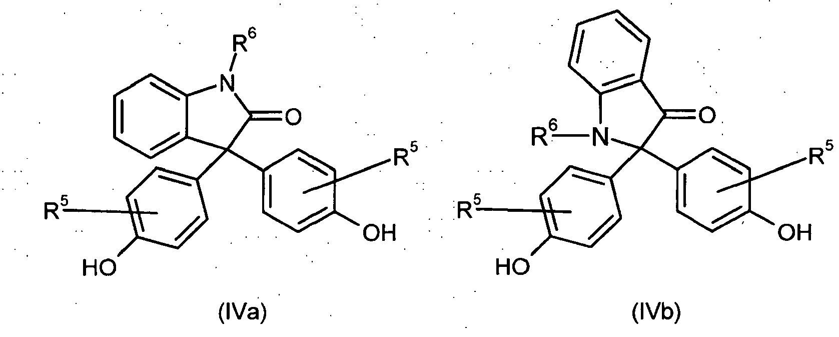 worin
R⁵ unabhängig voneinander für Wasserstoff oder C₁-C₁₀ Alkyl, bevorzugt für Wasserstoff oder C₁-C₆ Alkyl, besonders bevorzugt Wasserstoff oder C₁-C₄ Alkyl, ganz besonders bevorzugt für Wasserstoff oder Methyl steht
R⁶ für C₁-C₁₀ Alkyl, bevorzugt C₁-C₆ Alkyl, besonders bevorzugt C₁-C₄ Alkyl, jeweils gegebenenfalls substituiertes Phenyl oder Benzyl, insbesondere Methyl, Phenyl oder Benzyl steht, wobei als Substituenten für Phenyl und Benzyl die bei R¹ genannten Reste bevorzugt sind.

3. Mehrschichtiges Erzeugnis gemäß Anspruch 2, **dadurch gekennzeichnet, dass** die (Co)Polycarbonat(e) 20 bis 100 Mol% Diphenol der Formel (I) und 80 bis 0 Mol-% (jeweils bezogen auf die Menge eingesetzter Diphenole) Diphenole der Formel (II) und / oder (IV) enthalten.

4. Mehrschichtiges Erzeugnis gemäß Anspruch 2 oder 3, **dadurch gekennzeichnet, dass** Diphenole der Formel (II) ausgewählt sind aus mindestens einem aus der Gruppe bestehend aus Bisphenol A, 4,4'-Dihydroxybiphenyl, 2,2-Bis-(3-methyl-4-hydroxyphenyl)-propan, Bisphenol TMC.

5. Mehrschichtiges Erzeugnis gemäß einem der Ansprüche 1 bis 4, **dadurch gekennzeichnet, dass** das (Co)Polycarbonat oder das Blend ein oder mehrere Additive aus der Klasse der Thermostabilisatoren, Entformungsmittel, UV-Absorber und Füllstoffe enthält.

6. Mehrschichtiges Erzeugnis gemäß einem der Ansprüche 1 bis 5, **dadurch gekennzeichnet, dass** die Schicht auf der Substratschicht eine Metallschicht ist.

7. Mehrschichtiges Erzeugnis gemäß Anspruch 6, **dadurch gekennzeichnet, dass** auf der Metallschicht eine weitere Schutzschicht aufgetragen ist.

8. Verfahren zur Erzeugung eines mehrschichtigen Erzeugnisses gemäß Anspruch 7, **dadurch gekennzeichnet, dass** die Schutzschicht in einem PECVD oder Plasmapolymerisationsprozess aufgebracht wird.

9. Verfahren zur Erzeugung eines mehrschichtigen Erzeugnisses nach Anspruch 8, **dadurch gekennzeichnet, dass** die Erzeugung der Schutzschicht durch PECVD oder Plasmapolymerisationsprozess von einer oder mehreren leichtflüchtigen Komponenten ausgewählt aus Hexamethyldisiloxan (HMDSO), Hexamethyldisilazan (HMDS), Tetrametyldisiloxan, Decamethylcyclopentasiloxan, Octamethylcyclotetrasiloxan und Trimethoximethylsilan erfolgt.

## Claims

1. Multilayer product containing a substrate which has a further layer at least on one side, the substrate being produced from at least one (co)polycarbonate or at least one blend containing one or more (co)polycarbonates and one or more thermoplastic polymers, **characterized in that** the (co)polycarbonate(s) contain bisphenols of the formula (Ia) as a repeating monomer unit in the polymer

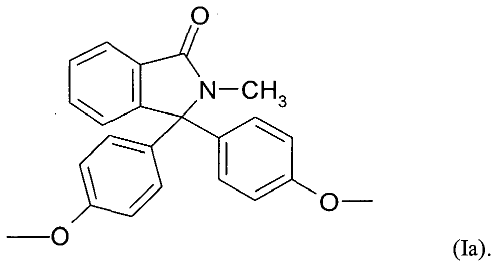

2. Multilayer product according to Claim 1, **characterized in that** the (co)polycarbonate(s) contain up to 95 mol% (based on the amount of diphenols used) of one or more diphenols selected from diphenols of the formula (II) and diphenols of the formula (IV), it being possible for diphenols of the formula (IV) to be an isomer mixture of diphenols of the formulae (IVa) and (IVb) 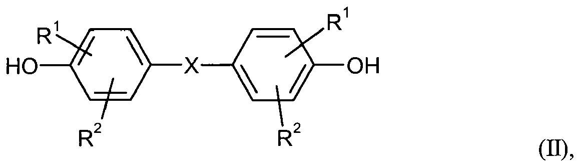 in which
R¹ and R², independently of one another, represent hydrogen, C₁-C₁₈-alkyl, C₁-C₁₈-alkoxy, halogen, in each case optionally substituted aryl or aralkyl, and
X represents a single bond, -SO₂-, -CO-, -O-, -S-, C₁- to C₆-alkylene, C₂- to C₅-alkylidene or C₅- to C₆-cycloalkylidene, which may be substituted by C₁- to C₆-alkyl, or represents C₆-C₁₂-arylene, which may optionally be condensed with further aromatic rings containing heteroatoms, and/or 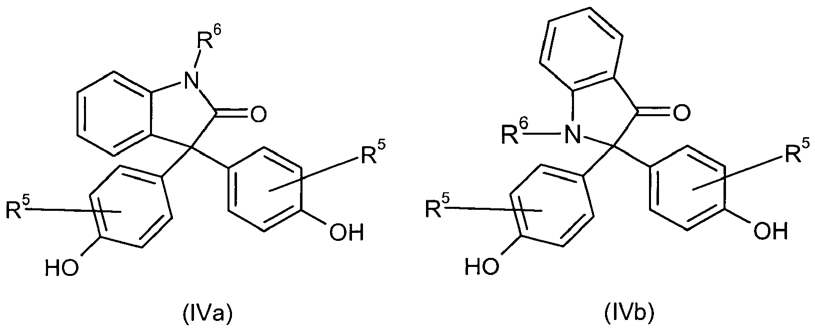 in which
R⁵ independently of one another; represents hydrogen or C₁-C₁₀ alkyl, preferably hydrogen or C₁-C₆ alkyl, particularly preferably hydrogen or C₁-C₄ alkyl, very particularly preferably hydrogen or methyl
R⁶ represents C₁-C₁₀ alkyl, preferably C₁-C₆ alkyl, particularly preferably C₁-C₄ alkyl, in each case optionally substituted phenyl or benzyl, in particular methyl, phenyl or benzyl, the radicals mentioned in the case of R¹ being preferred as substituents for phenyl and benzyl.

3. Multilayer product according to Claim 2, **characterized in that** the (co)polycarbonate(s) contain 20 to 100 mol% of diphenol of the formula (I) and 80 to 0 mol% (in each case based on the amount of diphenols used) of diphenols of the formula (II) and/or (IV).

4. Multilayer product according to Claim 2 or 3, **characterized in that** diphenols of the formula (II) are selected from at least one from the group consisting of bisphenol A, 4,4'-dihydroxybiphenyl, 2,2-bis(3-methyl-4-hydroxyphenyl)propane, bisphenol TMC.

5. Multilayer product according to any of Claims 1 to 4, **characterized in that** the (co)polycarbonate or the blend contains one or more additives from the class consisting of the heat stabilizers, demoulding agents, UV absorbers and fillers.

6. Multilayer product according to any of Claims 1 to 5, **characterized in that** the layer on the substrate layer is a metal layer.

7. Multilayer product according to Claim 6, **characterized in that** a further protective layer is applied to the metal layer.

8. Process for the production of a multilayer product according to Claim 7, **characterized in that** the protective layer is applied in a PECVD or plasma polymerization process.

9. Process for the production of a multilayer product according to Claim 8, **characterized in that** the production of the protective layer is effected by PECVD or plasma polymerization process from one or more readily volatile components selected from hexamethyldisiloxane (HMDSO), hexamethyldisilazane (HMDS), tetramethyldisiloxane, decamethylcyclopentasiloxane, octamethylcyclotetrasiloxane and trimethoxymethylsilane.

## Revendications

1. Produit multicouche contenant un substrat, qui comprend une couche supplémentaire au moins sur un côté, le substrat étant fabriqué à partir d'au moins un (co)polycarbonate ou d'au moins un mélange contenant un ou plusieurs (co)polycarbonates et un ou plusieurs polymères thermoplastiques, **caractérisé en ce que** les (co)polycarbonates contiennent des bisphénols de formule (Ia) en tant qu'unité monomère de répétition dans le polymère

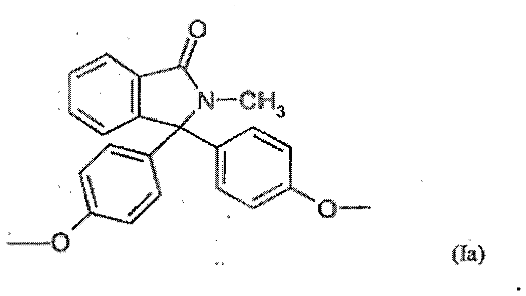

2. Produit multicouche selon la revendication 1, **caractérisé en ce que** les (co)polycarbonates contiennent jusqu'à 95 % en moles (par rapport à la quantité de diphénols utilisés) d'un ou de plusieurs diphénols, choisis parmi les diphénols de formule (II) et les diphénols de formule (IV), les diphénols de formule (IV) pouvant être un mélange d'isomères de diphénols de formule (IVa) et (IVb) 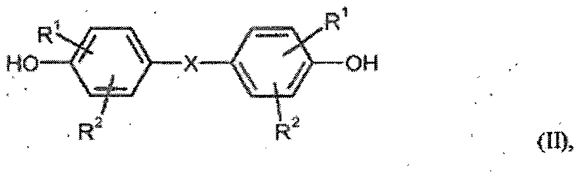 dans laquelle
R¹ et R² représentent indépendamment l'un de l'autre hydrogène, alkyle en C₁-C₁₈, alcoxy en C₁-C₁₈, halogène ; aryle ou aralkyle chacun éventuellement substitué, et
X représente une simple liaison, -SO₂-, -CO-, -O-, - S-, -alkylène en C₁ à C₆, alkylidène en C₂ à C₅ ou cycloalkylidène en C₅ à C₆, qui peut être substitué avec alkyle en C₁ à C₆ ; arylène en C₆-C₁₂, qui peut éventuellement être condensé avec des cycles aromatiques contenant des hétéroatomes supplémentaires, et/ou 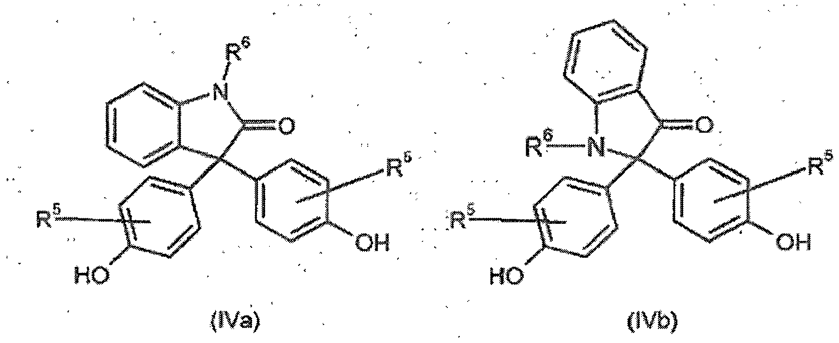
dans lesquelles
les R⁵ représentent indépendamment les uns des autres hydrogène ou alkyle en C₁-C₁₀, de préférence hydrogène ou alkyle en C₁-C₆, de manière particulièrement préférée hydrogène ou alkyle en C₁-C₄, de manière tout particulièrement préférée hydrogène ou méthyle,
R⁶ représente alkyle en C₁-C₁₀, de préférence alkyle en C₁-C₆, de manière particulièrement préférée alkyle en C₁-C₄ ; phényle ou benzène chacun éventuellement substitué ; notamment méthyle, phényle ou benzyle, les radicaux cités pour R¹ étant préférés en tant que substituants pour phényle et benzyle.

3. Produit multicouche selon la revendication 2, **caractérisé en ce que** les (co)polycarbonates contiennent 20 à 100 % en moles de diphénol de formule (I) et 80 à 0 % en moles (à chaque fois par rapport à la quantité de diphénols utilisés) de diphénols de formule (II) et/ou (IV).

4. Produit multicouche selon la revendication 2 ou 3, **caractérisé en ce que** les diphénols de formule (II) sont choisis parmi au moins un du groupe constitué par le bisphénol A, le 4,4'-dihydroxybiphényle, le 2,2-bis-(3-méthyl-4-hydroxyphényl)-propane, le bisphénol TMC.

5. Produit multicouche selon l'une quelconque des revendications 1 à 4, **caractérisé en ce que** le (co)polycarbonate ou le mélange contient un ou plusieurs additifs de la classe des stabilisateurs thermiques, des agents de démoulage, des absorbeurs UV et des charges.

6. Produit multicouche selon l'une quelconque des revendications 1 à 5, **caractérisé en ce que** la couche sur la couche de substrat est une couche métallique.

7. Produit multicouche selon la revendication 6, **caractérisé en ce qu'**une couche de protection supplémentaire est appliquée sur la couche métallique.

8. Procédé de formation d'un produit multicouche selon la revendication 7, **caractérisé en ce que** la couche de protection est appliquée par PECVD ou par un procédé de polymérisation plasma.

9. Procédé de formation d'un produit multicouche selon la revendication 8, **caractérisé en ce que** la formation de la couche de protection a lieu par PECVD ou par un procédé de polymérisation plasma d'un ou de plusieurs composants volatils choisis parmi l'hexaméthyldisiloxane (HMDSO), l'hexaméthyldisilazane (HMDS), le tétraméthyldisiloxane, le décaméthylcyclopentasiloxane, l'octaméthylcyclotétrasiloxane et le triméthoxyméthylsilane.
